# NEUE EUROPÄISCHE PATENTSCHRIFT

(11) **EP 0 881 906 B2**
(45) Veröffentlichungstag und Bekanntmachung des Hinweises auf die Entscheidung über den Einspruch: **04.02.2009**
(45) Hinweis auf die Patenterteilung: 25.08.2004
(21) Anmeldenummer: 97905068.9
(22) Anmeldetag: 21.02.1997
(51) Int. Cl.: A61K 38/02, A61K 38/19, A61K 39/39, A61K 47/00

(54) **PHARMAZEUTISCHE ZUSAMMENSETZUNG FÜR DIE IMMUNMODULATION BERUHEND AUF PEPTIDEN UND ADJUVANTIEN**
PHARMACEUTICAL COMPOSITION FOR IMMUNOMODULATION BASED ON PEPTIDES AND ADJUVANTS
COMPOSITION PHARMACEUTIQUE POUR L'IMMUNOMODULATION A BASE DE PEPTIDES ET D'ADJUVANTS

(30) Priorität: 24.02.1996 DE 19607044; 19.09.1996 DE 19638313; 25.11.1996 DE 19648687
(43) Veröffentlichungstag der Anmeldung: 09.12.1998
(73) Patentinhaber: Boehringer Ingelheim International GmbH, 55218 Ingelheim am Rhein (DE)
(72) Erfinder: SCHMIDT, Walter, A-1030 Wien (AT); BIRNSTIEL, Max, A-1080 Wien (AT); STEINLEIN, Peter, A-1020 Wien (AT); BUSCHLE, Michael, A-2345 Brunn/Gebirge (AT); SCHWEIGHOFFER, Tamàs, A-1180 Wien (AT)
(86) Internationale Anmeldenummer: PCT/EP1997/000828
(87) Internationale Veröffentlichungsnummer: WO 1997/030721

(56) Entgegenhaltungen:
- WO- -97/19169
- WO-A-95/02398
- GB-A- 2 191 494
- NATO ASI SER., SER. A (1991), 215(VACCINES), 25-32 CODEN: NALSDJ, 1991, XP000654387 BOMFORD, R. ET AL: "Immunomodulation by adjuvants"
- PROC. NATL. ACAD. SCI. U. S. A. (1996), 93(18), 9759-9763 CODEN: PNASA6;ISSN: 0027-8424, 1996, XP002033315 SCHMIDT, WALTER ET AL: "Transloading of tumor cells with foreign major histocompatibility complex class I peptide ligand: a novel general strategy for the generation of potent cancer vaccines"
- EUR. J. IMMUNOL. (1994), 24(3), 765-8 CODEN: EJIMAF;ISSN: 0014-2980, 1994, XP000654445 STUBER, GYOERGY ET AL: "Identification of wild-type and mutant p53 peptides binding to HLA-A2 assessed by a peptide loading-deficient cell line assay and a novel major histocompatibility complex class I peptide binding assay"
- GUPTA ET AL: 'Adjuvants for human vaccines - current status, problems and future prospects' VACCINE Bd. 13, Nr. 14, 1995 - 1995, Seiten 1263 - 1276
- WALKER ET AL.: 'Cationic lipids direct a viral glycoprotein into the class I major histocompatibility complex antigen-presentation pathway' PROC.NATL.ACAD.SCI. Bd. 89, September 1992 - September 1992, USA, Seiten 7915 - 7918
- BUSCHLE ET AL.: 'Transloading of tumor antigen-derived peptides into antigen-presenting cells' PROC.NATL.ACAD.SCI. Bd. 94, April 1997 - April 1997, USA, Seiten 3256 - 3261

## Beschreibung

Die Erfindung bezieht sich auf das Gebiet der Immunmodulation.

Die Erfindung ist eine Weiterentwicklung einer therapeutischen Vakzine auf der Grundlage von Tumorzellen, die im wesentlichen auf den folgenden Voraussetzungen beruht: es bestehen qualitative oder quantitative Unterschiede zwischen Tumorzellen und normalen Zellen; das Immunsystem hat prinzipiell die Fähigkeit, diese Unterschiede zu erkennen; das Immunsystem kann - durch aktive spezifische Immunisierung mit Vakzinen - dazu stimuliert werden, Tumorzellen anhand dieser Unterschiede zu erkennen und deren Abstoßung herbeizuführen.

Um eine Anti-Tumorantwort herbeizuführen, müssen zumindest zwei Voraussetzungen erfüllt sein: erstens müssen die Tumorzellen Antigene exprimieren, die auf normalen Zellen nicht oder nur derart begrenzt vorkommen, daß eine qualitative Unterscheidung zwischen Normal- und Tumorgewebe durch das Immunsystem möglich ist. Zweitens muß das Immunsystem entsprechend aktiviert werden, um auf diese Antigene zu reagieren. Ein wesentliches Hindernis bei der Immuntherapie von Tumoren ist deren geringe Immunogenität, vor allem im Menschen.
In jüngerer Zeit wurden Tumor-assoziierte und Tumor-spezifische Antigene entdeckt, die solche Neo-Epitope darstellen und somit potentielle Ziele für einen Angriff des Immunsystems sein sollten. Daß es dem Immunsystem dennoch nicht gelingt, Tumoren zu eliminieren, die diese Neo-Epitope exprimieren, dürfte demnach offensichtlich nicht am Fehlen von Neo-Epitopen gelegen sein, sondern daran, daß die immunologische Antwort auf diese Neo-Antigene unzureichend ist.

Für die Immuntherapie von Krebs auf zellulärer Basis wurden zwei allgemeine Strategien entwickelt: Einerseits die adoptive Immuntherapie, die sich der in vitro Expansion von tumorreaktiven T-Lymphozyten und deren Wiedereinführung in den Patienten bedient; andererseits die aktive Immuntherapie, welche Tumorzellen verwendet, in der Erwartung, daß damit entweder neue oder verstärkte Immunantworten gegen Tumorantigene hervorgerufen werden, die zu einer systemischen Tumorantwort führen. Tumorvakzine auf der Grundlage der aktiven Immuntherapie wurden auf verschiedene Arten hergestellt; ein Beispiel dafür sind bestrahlte Tumorzellen, die mit immunstimulierenden Adjuvantien wie Corynebacterium parvum oder Bacillus Calmette Guerin (BCG) versetzt werden, um Immunreaktionen gegen Tumorantigene hervorzurufen (Oettgen und Old, 1991)."Immunomodulation by adjuvants", siehe NATO ASI SER. A, 215 (VACCINES), 25-32, 1991.

In den letzten Jahren wurden vor allem genetisch modifizierte Tumorzellen für eine aktive Immuntherapie gegen Krebs verwendet. Eine Übersicht über diese verschiedenen Ansätze, bei denen Tumorzellen im Hinblick auf eine verstärkte Immunogenität durch Einführung verschiedener Gene verfremdet werden, wird von Zatloukal et al., 1993, gegeben. Eine der bisher eingesetzten Strategien verwendet Tumorzellen, die genetisch modifiziert werden, um Zytokine zu produzieren.

Die Identifizierung und Isolierung von Tumorantigenen und Tumor-assoziierten Antigenen (TAs) bzw. davon abgleiteter Peptide (z.B. beschrieben von Wölfel et al., 1994 a) und 1994 b); Carrel et al., 1993, Lehmann et al., 1989, Tibbets et al., 1993, oder in den veröffentlichten internationalen Anmeldungen WO 92/20356, WO 94/05304, WO 94/23031, WO 95/00159 beschrieben), war die Voraussetzung für eine weitere Strategie, bei der Tumorantigene als Immunogene für Tumorvakzine verwendet werden, und zwar sowohl in Form von Proteinen als auch von Peptiden. Durch die Arbeiten von Boon et al., 1992; Boon et al.; 1994; Boon et al., 1995; van Peel et al., 1995; Van der Eynde, 1995; wurde bekannt, daß maligne Melanome Tumorantigen-abgeleitete Peptide im MHC-I-Kontext präsentieren. Mit einer Tumorvakzine in Form von Tumorantigenen als solchen wurde jedoch bisher keine ausreichende Immunogenität erreicht, um eine zelluläre Immunantwort auszulösen, wie sie zur Eliminierung von Tumorantigen tragenden Tumorzellen erforderlich wäre (Marchand et al., 1995). Um zu erreichen, daß Antigen präsentierende Zellen ("Antigenpresenting cells", "APCs") definierte Peptidantigene auf ihrer Oberfläche prasentieren, wurde vorgeschlagen, die Peptide zu "pulsen", was jedoch in einer meffizienten Beladung der Zellen mit Peptiden resultierte (Tykocinski et al., 1996); es wurde auch gezeigt, daß die co-Applikation von Adjuvantien die Immunantwort nur bedingt verstärkte (Oettgen und Old, 1991).

Eine dritte Strategie der aktiven Immuntherapie zur Steigerung der Wirksamkeit von Tumorvakzinen basiert auf xenogenisierten (verfremdeten) autologen Tumorzellen. Diesem Konzept liegt die Annahme zugrunde, daß das Immunsystem auf Tumorzellen reagiert, die ein Fremdprotein exprimieren und daß im Zuge dieser Reaktion auch eine Immunantwort gegen diejenigen Tumorantigene hervorgerufen wird, die von den Tumorzellen der Vakzine präsentiert werden.

Eine zentrale Rolle bei der Regulierung der spezifischen Immunantwort spielt ein trimolekularer Komplex, bestehend aus den Komponenten T-Zell-Antigenrezeptor, MHC ("Major Histocompatibility Complex")-Molekül und dessen Liganden, der ein von einem Protein abgeleitetes Peptidfragment ist.

MHC-Moleküle (bzw. die entsprechenden humanen Moleküle, die HLAs) sind Peptidrezeptoren, die bei stringenter Spezifität die Bindung zahlreicher verschiedener Liganden erlauben. Die Voraussetzung dafür stellen Allelspezifische Peptidmotive dar, die folgende Spezifitätskriterien aufweisen: Die Peptide haben, in Abhängigkeit vom MHC-Haplotyp, eine definierte Länge, beimMHC-I Haplotyp in der Regel acht bis zehn Aminosäurereste. Typischerweise stellen zwei der Aminosäurepositionen sog. "Anker" dar, die nur durch eine einzige Aminosäure oder durch Aminosäure-Reste mit eng verwandten Seitenketten besetzt werden können. Die genaue Lage der Ankeraminosäuren im Peptid und die Anforderungen an deren Eigenschaften variieren mit den MHC-Haplotypen. Der C-Terminus der Peptid-Liganden ist häufig ein aliphatischer oder ein geladener Rest. Solche MHC-I-Peptid-Ligandenmotive sind bisher u.a. für H-2K^{d}, K^{b}, K^{k}, K^{km1}, D^{b}, HLA-A*0201, A*0205 und B*2705 Haplotypen bekannt.

Im Rahmen des Proteinumsatzes innerhalb der Zelle werden reguläre, entartete und fremde Genprodukte, z.B. virale Proteine oder Tumorantigene, in kleine Peptide zerlegt; einige davon stellen potentielle Liganden für MHC-Moleküle dar. Damit ist die Voraussetzung für deren Präsentation durch MHC-Moleküle und als Folge davon die Auslösung einer zellulären Immununatwort gegeben, wobei noch nicht im einzelnen aufgeklärt ist, wie die Peptide als MHC-Liganden in der Zelle produziert werden. Fremde oder verfremdete Proteine und deren Bruchstücke können auch durch Immunglobuline, die die humorale Immunantwort darstellen, erkannt, gebunden und beseitigt werden. Das gilt auch für sämtliche Tumorantigene: am Beispiel der Tumor-assoziierten Antigene MUC1, CEA und HER2/neu hat man bewiesen, daß Immunglobuline, welche für diese Proteine Spezifität aufweisen, die proteintragenden Zellen erkennen und abtöten können. Um eine Tumorantigen-spezifische humorale Immunantwort auszulösen, wurden deshalb verschiedene Formen von MUC1 und CEA als Immunogene (z.B. in rekombinanten Poxvektoren; Bronte at al., J.Immunol. 154:5282 1995) in Tiermodellen und klinischen Vorversuchen erprobt.

Im Rahmen der vorliegenden Erfindung wurden Überlegungen weitergeführt, die bei der Entwicklung einer zellulären Tumorvakzine angestellt wurden: während nicht-maligne, normale Körperzellen vom Immunsystem toleriert werden, reagiert der Körper auf eine normale Zelle, wenn sie, z.B. aufgrund einer Virusinfektion, körperfremde Proteine synthetisiert, mit einer Immunabwehr. Die Ursache dafür ist darin gelegen, daß die MHC-Moleküle Fremdpeptide präsentieren, die von den körperfremden Proteinen stammen. Als Folge davon registriert das Immunsystem, daß etwas Unerwünschtes, Fremdes mit dieser Zelle geschehen ist. APCs (dazu zählen Makrophagen, dendritische Zellen, Langerhans-Zellen, B-Zellen sowie möglicherweise die kürzlich entdeckten biphänotypischen Zellen, die sowohl Eigenschaften von B-Zellen als auch von Makrophagen aufweisen; Tykocinski et al., 1996) werden aktiviert, eine neue, spezifische Immunität generiert und die Zelle eliminiert.

Tumorzellen enthalten zwar die jeweiligen tumorspezifischen Tumorantigene, sind aber als solche unzulängliche Vakzine, weil sie aufgrund ihrer geringen Immunogenität vom Immunsystem ignoriert werden. Belädt man nun eine Tumorzelle nicht mit einem Fremdprotein, sondern mit einem Fremdpeptid, so werden zusätzlich zu den Fremdpeptiden auch die zelleigenen Tumorantigene dieser Zelle als fremd wahrgenommen. Durch die Verfremdung mit einem Peptid kann erreicht werden, daß sich die durch die Fremdpeptide ausgelöste zelluläre Immunantwort gegen die Tumorantigene richtet.

Die Ursache für die geringe Immunogenität von Tumorzellen kann nicht nur ein qualitatives, sondern ein quantitatives Problem sein. Für ein von einem Tumorantigen abgeleitetes Peptid kann das bedeuten, daß es zwar von MHC-Molekülen präsentiert wird, jedoch in einer Konzentration, die zu gering ist, um eine zelluläre tumorspezifische Immunantwort auszulösen. Eine Erhöhung der Zahl von tumorspezifischen Peptiden auf der Tumorzelle sollte somit ebenfalls eine Verfremdung der Tumorzelle bewirken, die zur Auslösung einer zellulären Immunantwort führt. Es wurde vorgeschlagen, das Tumorantigen bzw. das davon abgeleitete Peptid dadurch auf der Zelloberfläche zu präsentieren, daß es mit einer für das betreffende Protein bzw. Peptid kodierenden DNA transfiziert wird, wie in den internationalen Veröffentlichungen WO 92/20356, WO 94/05304, WO 94/23031 und WO 95/00159, beschrieben.

In der deutschen Patentanmeldung P 195 43 649.0 ist eine zelluläre Vakzine geoffenbart, die als aktive Komponente Tumorzellen enthält, die mit einem oder mehreren Peptiden derart beladen sind, daß die Tumorzellen im Kontext mit den Peptiden vom Immunsystem des Patienten als fremd erkannt werden und eine zelluläre Immunantwort auslösen. Ein wesentliches Merkmal der Peptide ist, daß sie Liganden für den MHC-Haplotyp des Patienten sind. Die Peptide werden deshalb vom Immunsystem des Patienten als fremd erkannt, weil sie einerseits "Fremdpeptide" oder "Xenopeptide" sein können, d.h. sie sind verschieden von Peptiden, die abgeleitet von Proteinen sind, die von Tumorzellen des Patienten exprimiert werden. Eine andere Kategorie von Peptiden ist von Tumorantigenen abgeleitet, die von Zellen des Patienten exprimiert werden. Diese bewirken eine Steigerung der Immunogenität dadurch, daß sie in einer Konzentration auf den Tumorzellen der Vakzine vorliegen, die höher ist als die Konzentration desselben Peptids auf den Tumorzellen des Patienten.

Der vorliegenden Erfindung lag die Aufgabe zugrunde, eine neue immunmodulatorisch wirkende pharmazeutische Zusammensetzung, insbesondere eine Vakzine, bereitzustellen.

In Weiterführung des Konzepts der in der deutschen Patentanmeldung P 195 43 649.0 geoffenbarten zellulären Vakzine wurde im Rahmen der vorliegenden Erfindung eine pharmazeutische Zusammensetzung entwickelt, die immunmodulatorisch wirkende Peptide nicht im Kontext mit Zellen, sondern zusammen mit einem Adjuvans enthält, um eine zelluläre und/oder humorale, vorzugsweise eine systemische, Immunantwort gegen pathogene Erreger bzw. eine Anti-Tumorantwort auszulösen bzw. zu verstärken oder eine Toleranz gegen autoimmun wirkende Proteine hervorzurufen.

Es wurde überraschend festgestellt, daß bestimmte Adjuvantien, z.B. Polykationen, von denen erstmals bereits 1965 gezeigt wurde, daß sie eine Steigerung des Transports von Proteinen in Zellen bewirken können (Ryser et al., 1965; Ryser et al., 1978; Shen et al., 1981) die Immunogenität von Peptiden steigern.

Die Erfindung betrifft eine pharmazeutische Zusammensetzung die in den Patentansprüchen 1 bis 14 charakterisiert ist. Die Zusammensetzung ist dadurch gekennzeichnet, daß das Adjuvans die Fähigkeit aufweist, die Bindung des Peptids bzw. des Proteins oder Proteinfragments an APC Zellen des zu behandelnden Individuums bzw. den Eintritt in die Zellen zu steigern und eine Verstärkung der immunmodulatorischen Wirkung des Peptids bzw. des Proteins oder Proteinfragments zu bewirken.

Im folgenden werden im allgemeinen, der Einfachheit halber, Vertreter der genannten immunmodulatorisch wirkenden Peptide, Proteine oder Proteinfragmente, als "Peptide" bezeichnet. Der Begriff "Peptid" steht, wenn er sich nicht ausdrücklich auf den Liganden bezieht, auch stellvertretend für größere Proteinfragmente oder Proteine, von denen das Peptid abgeleitet ist bzw. ein zelluläres Abbauprodukt darstellt.

Unter "immunmodulatorischer Wirkung" wird einerseits die Auslösung oder Verstärkung einer zellulären und/oder humoralen, vorzugsweise systemischen Immunreaktion verstanden. In dieser Ausführungsform wirkt die erfindungsgemäße pharmazeutische Zusammensetzung als Vakzine.

In einer bevorzugten Ausführungsform sind die Peptide Liganden für mindestens ein MHC-Molekül, das vom zu behandelnden Individuum exprimiert wird.

Die humanen MHC-Moleküle werden, gemäß den internationalen Gepflogenheiten, im folgenden auch als "HLA" ("Human Leucocyte Antigen") bezeichnet.

Unter "zelluläre Immunantwort" ist vor allem die zytotoxische T-Zellimmunität zu verstehen, die als Folge der Generierung von zytotoxischen CD8-positiven T-Zellen und CD4-positiven Helfer-T-Zellen die Zerstörung der vom pathogenen Erreger befallenen Zellen bewirkt.

Unter "humorale Immunantwort" ist die Produktion von Imunglobulinen zu verstehen, die selektiv von pathogenen Erregern abgeleitete Strukturen erkennen und in der Folge zusammen mit anderen Systemen, wie z.B. Komplement, ADCC (Antibody dependent Cytotoxicity) oder Phagozytose, die Zerstörung der pathogenen Erreger oder der davon befallenen Zellen bewirken.

Das in der Vakzine enthaltene Peptid ist abgeleitet von einem Antigen bzw. stellt im Fall von Proteinen ein Antigen dar, gegen das eine zelluläre und/oder humorale Immunantwort ausgelöst werden soll. Damit wird bewirkt, daß T-Zellen bzw. andere zytotoxische Effektorzellen, die den Krankheitserreger die das Antigen aufweisen, erkennen, und/oder Antikörper generiert werden.

Für die Immunisierung gegen Krankheitserreger, wie Bakterien, Viren, Parasiten, werden Proteine bzw. Peptide verwendet, die ein Protein des bzw. der jeweiligen Erreger darstellen bzw. davon abgeleitet sind. Geeignet sind dabei vor allem Proteine, die von der hohen allgemeinen Mutationsrate dieser Erreger veschont bleiben. Publizierte Beispiele sind HPV16/17 (Human Papilloma Virus; Feltkamp et al., 1995), Hepatitis B Virus Core Antigen (Vitiello et al., 1995), Plasmodium Bergheii (Widmann et al., 1992.), Influenzavirus-Nukleoprotein, Hepatitis C Virus.

In einer Ausführungsform der Erfindung dient die pharmazeutische Zusammensetzung dazu, eine Toleranz gegen Proteine bzw. deren Fragmente herbeizuführen, die autoimmun induzierte Erkrankungen auslösen, also zur Therapie von Autoimmunerkrankungen. Die in dieser Ausführungsform der Erfindung verwendeten Peptide sind von Proteinen abgeleitet, die Autoimmunerkrankungen verursachen.

Im Gegensatz zu der Anwendung als als Vakzine gegen pathogene Erreger, bei der die Peptide mit einem Abschnitt des Ursprungproteins (Protein des Pathogens) im wesentlichen derart übereinstimmen, daß das Peptid als das "Original-Antigen" erkannt wird, werden bei der Anwendung der Erfindung zur Behandlung von Autoimmunerkrankungen u.a. Peptide verwendet, die zur Aminosäuresequenz des Ursprungproteins kritische Unterschiede aufweisen. Diese Peptide binden zwar aufgrund ihrer Ankerpositionen an das MHC-Molekül, weisen jedoch in ihrer Sequenz Mutationen auf, die bewirken, daß diese Peptide als Antagonisten funktionieren, welche die aktivierten, spezifischen T-Zellen wieder abschalten (Kersh und Allen, 1996).

Als Peptid-Antagonisten eignen sich sowohl "natürliche" Antagonisten, die in Viren entdeckt wurden (Bertoletti et al., 1994), als auch Antagonisten, die durch systematische Forschung, z.B. durch Screenen von Peptid-Libraries, gefunden wurden. Ein Beispiel für Peptid-Antagonisten sind Peptide, die T-Zellen ausschalten können, welche spezifisch für Myelin Basic Protein sind, diese wurden in Tierexperimenten auf ihre Wirksamkeit erprobt. (Brocke et al., 1996).

Ein Peptid, das eine zelluläre Immunantwort auslösen soll, muß an ein MHC-Molekül binden können. Damit die Immunantwort im Patienten ausgelöst wird, muß somit das zu behandelnde Individuum ein entsprechendes HLA-Molekül in seinem Repertoire aufweisen. Die Bestimmung des HLA-Subtyps des Patienten stellt somit, im Hinblick auf die Auslösung einer zellulären Immunantwort, eine der wesentlichen Voraussetzungen für die wirksame Anwendung eines Peptids an diesem Patienten dar.

Der HLA-Subtyp des Patienten kann mit Standardmethoden, wie dem Mikro-Lymphotoxizitätstest bestimmt werden (Practical lmmunology., 1989). Dieser Test beruht auf dem Prinzip, aus Patientenblut isolierte Lymphozyten zunächst mit Antiserum oder einem monoklonalen Antikörper gegen ein bestimmtes HLA-Molekül in Gegenwart von Kaninchen-Komplement (C) zu versetzen. Positive Zellen werden lysiert und nehmen einen IndikatorFarbstoff auf, während unbeschädigte Zellen ungefärbt bleiben.

Zur Bestimmung des HLA-I- oder HLA-II-Haplotyps eines Patienten kann auch die RT-PCR ("Reverse Transcriptase Polymerase Chain Reaction") herangezogen werden (Curr. Prot. Mol. Biol. Kapitel 2 und 15). Dazu entnimmt man einem Patienten Blut und isoliert daraus RNA. Diese RNA unterwirft man zunächst einer Reversen Transkription, wodurch cDNA des Patienten entsteht. Die cDNA dient als Matrize für die Polymerasekettenreaktion mit Primerpaaren, die spezifisch die Amptifikation eines DNA-Fragmentes bewirken, das für einen bestimmten HLA-Haplotyp steht. Erscheint nach Agarosegelelektrophorese eine DNA-Bande, exprimiert der Patient das entsprechende HLA-Molekül. Erscheint die Bande nicht, ist der Patient dafür negativ.

Die Definition eines erfindungsgemäß verwendeten Peptids durch ein HLA-Molekül bestimmt dieses hinsichtlich seiner Ankeraminosäuren und seiner Länge; definierte Ankerpositionen und Länge gewährleisten, daß ein Peptid in die Peptid-Bindungsfurche des jeweiligen HLA-Moleküls des Patienten paßt. Dies hat zur Folge, daß das Immunsystem stimuliert wird und eine zelluläre Immunreaktion erzeugt wird, die sich, im Falle der Verwendung eines von einem Tumorantigen abgeleiteten Peptids, gegen die Tumorzellen des Patienten richtet.

Peptide, die zur Anwendung im Rahmen der vorliegenden Erfindung geeignet sind, sind in einer großen Bandbreite verfügbar. Ihre Sequenz kann von natürlich vorkommenden immunogenen Proteinen bzw. deren zellulären Abbauprodukten, z.B. von viralen oder bakteriellen Peptiden, von Tumorantigenen, abgeleitet sein, oder sie können Antagonisten zu Peptiden sein, die von Autoimmunerkrankungen induzierenden Proteinen abgeleitet sind.

Geeignete Peptide können z.B. auf der Grundlage von literaturbekannten Peptidsequenzen ausgewählt werden.

Im Hinblick auf die Auslösung einer zellulären Immunantwort können die Peptide z.B. anhand der von Rammensee et. al., 1993, Rammensee et al., 1995, Falk et al., 1991, für die unterschiedlichen HLA-Motive beschriebenen, von immunogenen Proteinen verschiedenen Ursprungs abgeleiteten Peptide definiert werden, die in die Bindungsfurchen der Moleküle der jeweiligen HLA-Subtypen passen.

Für Peptide, die eine Teilsequenz eines Proteins mit immunogener Wirkung aufweisen, kann anhand der bereits bekannten oder gegebenfalls noch zu bestimmenden Polypeptidsequenzen durch Sequenzabgleich unter Berücksichtigung der HLA-spezifischen Anforderungen festgestellt werden, welche Peptide geeignete Kandidaten darstellen. Beispiele für geeignete Peptide finden sich z.B. bei Rammensee et al., 1993, Falk et al., 1991, und Rammensee, 1995; sowie in der WO 91/09869 (HIV-Peptide); von Tumorantigenen abgeleitete Peptide wurden u.a. in den veröffentlichten internationalen Patentanmeldungen WO 95/00159, WO 94/05304 beschrieben. Auf die Offenbarung dieser Literaturstellen und der darin im Zusammenhang mit Peptiden zitierten Artikel wird beispielhaft Bezug genommen. Zu bevorzugten Kandidaten zählen Peptide, deren Immunogenität bereits gezeigt wurde, also Peptide, die von bekannten Immunogenen, z.B. viralen oder bakteriellen Proteinen, abgeleitet sind.

Statt die Originalpeptide zu verwenden, die in die Bindungsfurche von MHC-Ioder MHC-II-Molekülen passen, also Peptide, die unverändert von natürlichen Proteinen abgeleitet sind, können anhand der auf der Grundlage der Originalpeptidsequenz angegebenen Minimalanforderungen bezüglich Ankerpositionen und Länge Variationen vorgenommen werden, sofern durch diese Variationen die effektive Immunogenität des Peptids, die sich zusammensetzt aus seiner Bindungsaffinität an das MHC-Molekül und seiner Fähigkeit, T-Zell-Rezeptoren zu stimulieren, nicht nur nicht beeinträchtigt ist, sondern vorzugsweise verstärkt wird. In diesem Fall werden also erfindungsgemäß künstliche Peptide verwendet, die entsprechend den Anforderungen der Bindungsfähigkeit an ein MHC-Molekül entworfen sind.
So können z.B. ausgehend vom H2-K^{d}-Liganden Leu Phe Glu Ala Ile Glu Gly Phe Ile (LFEAIEGFI) die Aminosäuren, die keine Ankeraminosäuren darstellen, geändert werden, um das Peptid der Sequenz Phe Phe Ile Gly Ala Leu Glu Glu Ile (FFIGALEEI) zu erhalten; außerdem kann die Ankeraminosäure Ile an Position 9 durch Leu ersetzt werden. Die Bestimmung von Epitopen von MHC-I- bzw. MHC-II-Liganden bzw. deren Variation kann z.B. nach dem von Rammensee et al., 1995, beschriebenen Prinzip vorgenommen werden. Die Länge des Peptids entspricht im Falle seiner Abstimmung auf MHC-I Moleküle vorzugsweise einer Minimalsequenz von 8 bis 10 Aminosäuren mit den erforderlichen Ankeraminosäuren. Das MHC-II-Bindungsmotiv, das sich über neuen Aminosäuren erstreckt, weist einen höheren Grad an Degeneration in den Ankerpositionen auf. Es wurden kürzlich, ausgehend von der Röntgenstrukturanalyse von MHC-II-Molekülen, Methoden entwickelt, die die genaue Analyse der MHC-II-Bindungsmotive, und ausgehend davon, Variationen der Peptidsequenz erlauben (Rammensee et al., 1995, und die dort zitierte Originalliteratur).

Gegebenenfalls kann das Peptid auch am C- und/oder am N-Terminus verlängert sein, sofern diese Verlängerung die Bindungsfähigkeit. an das MHC-Molekül nicht beeinträchtigt bzw. das verlängerte Peptid auf die Minimalsequenz zellulär prozessiert werden kann.

In einer Ausführungsform der Erfindung ist das Peptid negativ geladen. In dieser Ausführungsform kann das Peptid mit negativ geladenen Aminosäuren verlängert werden, oder es können negativ geladene Aminosäuren in das Peptid, vorzugsweise an Positionen, die für die Erkennung durch spezifische CTLs oder als Ankeraminosäuren nicht erforderlich sind, eingebaut werden, um eine elektrostatische Bindung des Peptids an ein polykationisches Adjuvans, wie Polylysin, zu erreichen.
In einer Ausführungsform der Erfindung wird das Antigen nicht in Form eines Peptids, sondern als Protein oder Proteinfragment bzw. als Gemisch von Proteinen oder Proteinfragmenten eingesetzt. Im Rahmen der vorliegenden Erfindung sind größere Proteinfragmente bzw, ganze Proteine geeignet, von denen gewährleistet ist, daß sie nach der Applikation von den APCs des Patienten zu Peptiden prozessiert werden, die an das MHC-Molekül passen.

Das Protein stellt ein Antigen dar, von dem die nach Prozessierung erhaltenen Bruchstücke abgeleitet sind. In dieser. Ausführungsform dient das Adjuvans dazu, die Beladung ("Transloading") von Zellen, insbesondere APCs wie dendritischen Zellen oder Makrophagen, mit dem bzw. den Fragmenten zu ermöglichen oder zu verstärken. So aufgenommene Proteine bzw. Proteinfragmente werden von den Zellen prozessiert und können danach im MHC-Kontext den Immuneffektorzellen präsentiert werden und somit eine Immunantwort auslösen bzw. verstärken (Braciale und Braciale, 1991; Kovacsovics Bankowski und Rock, 1995; York und Rock, 1996).

Die Ausführungsform der Erfindung, in der Proteine bzw. größere Proteinfragmente als Antigene eingesetzt werden, hat den Vorteil, daß eine geringere Abhängigkeit vom HLA-Typ des Patienten besteht, weil das Protein in mehrere Bruchstücke prozessiert wird und somit eine größere Variabilität hinsichtlich der "Paßform" der Peptide gegeben ist.

Im Fall der Verabreichung von Proteinen oder Proteinfragmenten kann man die Identität der prozessierten Endprodukte mittels chemischer Analyse (Edman-Abbau oder Massenspektrometrie von prozessierten Fragmenten; vgl. den Übersichtsartikel von Rammensee et al., 1995 sowie die darin zitierte Originalliteratur) oder biologischen Assays (Fähigkeit der APCs zur Stimulation von T-Zellen, die für die prozessierten Fragmente spezifisch sind), nachweisen.

Die Auswahl von Peptid-Kandidaten, die geeignet sind, eine zelluläre Immunantwort auszulösen, erfolgt prinzipiell in mehreren Stufen: Im allgemeinen werden die Kandidaten, zweckmäßig in Serienversuchen, zunächst in einem Peptid-Bindungstest auf ihre Bindungsfähigkeit an ein MHC-Molekül getestet.

Eine dafür geeignete Untersuchungsmethode beruht auf der Fähigkeit von Peptiden, leere MHC-Moleküle stabilisieren zu können, wie z.B. von Stuber et al., 1994, und Mclntyre et al., 1996, beschrieben. Dabei wird das Peptid auf Zellen aufgebracht, die in der Lage sind, das jeweilige MHC-Molekül zu exprimieren, aber wegen eines genetischen Defekts keine endogenen Peptide im MHC-Kontext binden. Geeignete Zellinien dieses Typs sind RMA-S (Maus) und T2 (human), bzw. deren transfizierte Varianten. Es sind dann nur die vom jeweiligen Peptid stabilisierten MHC-Moleküle nachweisbar, vorzugsweise mittels der auf Durchflußzytometrie beruhenden FACS-Analyse (Flow Cytometry, 1989; FACS Vantage TM User's Guide, 1994; CELL Quest^{TM} User's Guide, 1994). Stabile MHC-Moleküle werden mit einem geeigneten anti-MHC-Antikörper und mit einem mit Fluoreszenzfarbstoff, z.B. FITC (Fluoresceinisothiocyanat) markierten zweiten (z.B. polyklonalen) Antikörper nachgewiesen. Im Durchfluß werden dann einzelne Zellen von einem Laser einer bestimmten Wellenlänge angeregt; die emittierte Fluoreszenz wird gemessen, sie ist abhängig von der an die Zelle gebundenen Peptidmenge.

Eine weitere Methode zur Bestimmung der gebundenen Peptidmenge ist der Scatchard-Plot, wie beschrieben von Sette et al., 1994. Man benutzt dazu das Peptid, das z.B. mit |¹²⁵ markiert ist, und inkubiert es über Nacht mit isolierten oder rekombinant hergestellten MHC-Molekülen bei 4°C mit verschiedenen definierten Konzentrationen von Peptid. Zur Bestimmung unspezifischer Wechselwirkung des Peptids wird zu einigen Proben ein Überschuß nicht-markierten Peptids zugesetzt, der die unspezifische Interaktion des markierten Peptids unterbindet. Anschließend entfernt man das unspezifisch gebundene Peptid, z.B. mittels Gelchromatographie. Die Menge des gebundenen Peptids wird nun in einem Szintillationszähler anhand der emittierten Radioaktivität ermittelt. Die Auswertung der so gewonnenen Daten erfolgt nach Standardmethoden.

Eine Übersicht über Methoden zur Charakterisierung der MHC/Peptid-Wechselwirkung, der Analyse von MHC-Liganden und Peptid-Bindungs-Assays, die im Rahmen der vorliegenden Erfindung verwendet werden können, wurde von Rammensee et al., 1995, gegeben.

In einem nächsten Schritt werden Peptid-Kandidaten mit guten Bindungsqualitäten auf ihre Immunogenität geprüft:

Die Auslösung einer zellulären Immunantwort kann durch den Nachweis peptidspezifischer CTLs bestätigt werden, z.B. mittels der in Current Protocols in Immunology, Kapitel 3, oder von Blomberg et al., 1993, beschriebenen Methode. Ein weiterer Nachweis für das Vorliegen einer zellulären Immunantwort ist dann gegeben, wenn in Abwesenheit von T-Zellen in einem Versuchstier (welche dadurch erzielt wird, daß man das Tier mit Antikörpern behandelt, die CD4- oder CD8-Zellen depletieren) keine Immunantwort auftritt (Current Protocols in Immunology, Kapitel 3).

Eine zelluläre Immunantwort kann auch durch den Nachweis einer "delayed-type hypersensitivity" (DTH)-Reaktion in immunisierten Tieren gezeigt werden. Hierzu können Peptide in die Fußsohle von Mäusen injiziert werden, worauf die Anschwellung der injizierten Stelle gemessen wird (Grohman et al., 1995; Puccetti et al., 1994).

Die Induktion einer humoralen Immunantwort durch Peptide, die Fremdantigene für den Organismus sind bzw. Antigene, die vom zu behandelnden Organismus in geringer Konzentration exprimiert werden, kann durch Nachweis von spezifischen Antikörpern im Serum bestimmt werden. Eine geeignete Methode zur Antikörpertiterbestimmung im Serum ist der Enzyme Linked Immunoassay (ELISA). Dabei werden die spezifischen Antikörper nach Bindung an das zur Immunisierung verwendeten Peptids mit einer Farbreaktion nachgewiesen. Eine alternative Methode ist der Western Blot. Hierbei binden spezifische Serumantikörper an das auf einer Membran immobilisierte Peptid. Gebundene Antikörper werden schließlich wiederum mit einer Farbreaktion nachgewiesen (Referenz für beide Methoden: Current Protocols in Immunology. Editors: Coligan et al.,1991,).

Vor allem nach der Vakzinierung mit Fremdantigenen, z.B. viralen Ursprungs, ist eine Bildung von Antikörpern zu erwarten.

Die Auslösung einer zellulären Immunantwort durch Peptide, die abgeleitet sind von Proteinen, deren immunogene Wirkung nicht bekannt ist, kann z.B. getestet werden, wie von Rivoltini et al. 1995, oder Kawakami et al., 1994a, beschrieben. Dazu benötigt man T-Zellen, die das gewünschte Peptid erkennen können, wenn es von MHC-Molekülen präsentiert wird. Im Fall von Fremdpeptiden gewinnt man solche T-Zellen analog aus dem peripheren Blut. Die T-Zellen werden mit Zellinien wie T2 (Alexander et al., 1989) oder RMA-S (Kärre et al., 1986), die mit dem jeweiligen Peptid versetzt worden sind, inkubiert und lysieren diese, falls es sich um ein immunogenes Peptid handelt.

Eine weitere Möglichkeit für die Testung von MHC-bindenden Peptidkandidaten auf ihre immunogenität besteht darin, die Bindung der Peptide an T2-Zellen zu untersuchen. Ein solcher Test beruht auf der Eigenart von T2-Zellen (Alexander et al., 1989) oder RMA-S-Zellen (Kärre et al., 1986), defekt im TAP-Peptid-Transportmechanismus zu sein und erst dann stabil MHC-Moleküle zu präsentieren, wenn man auf sie Peptide aufbringt, die im MHC-Kontext präsentiert werden. Für den Test werden z.B. T2-Zellen oder RMA-S-Zellen verwendet, die stabil mit einem HLA-Gen, z.B. mit HLA-A1- und/oder HLA-A2-Genen transfiziert sind. Werden die Zellen mit Peptiden versetzt, die gute HLA-Liganden sind, indem sie im HLA-Kontext so präsentiert werden, daß sie vom immunsystem als fremd erkannt werden können, bewirken solche Peptide, daß die HLA-Moleküle in signifikanter Menge auf der Zelloberfläche aufscheinen. Der Nachweis der HLAs auf der Zelloberfläche, z.B. mittels monoklonaler Antikörper, erlaubt die Identifizierung geeigneter Peptide (Malnati et al., 1995; Sykulev et al., 1994). Auch hier wird zweckmäßig ein Standardpeptid mit bekannt guter HLA-Bindungsfähigkeit verwendet.

Im Hinblick auf eine möglichst breite Anwendbarkeit der erfindungsgemäßen pharmazeutischen Zusammensetzung wird zweckmäßig eine Mischung mehrerer Peptide verwendet, von denen jedes an ein anderes MHC-Molekül binden kann, vorzugsweise an einen von zwei oder drei der am häufigsten vertretenen MHC-Subtypen. Mit einer Vakzine auf der Grundlage einer Mischung von Peptiden, die an diese Haplotypen binden können, kann eine breite Patientenpopulation erfaßt werden.

In einer Ausführungsform der Erfindung kann die Vakzine mehrere Peptide Sequenz aufweisen. Die verwendeten Peptide können sich in diesem Fall einerseits dadurch unterscheiden, daß sie an unterschiedliche HLA-Subtypen binden. Damit kann erreicht werden, daß mehrere bzw. sämtliche HLA-Subtypen eines Patienten oder einer größeren Gruppe von Patienten erfaßt werden.

Eine weitere, gegebenenfalls zusätzliche, Variabilität hinsichtlich der verwendeten Peptide kann darin bestehen, daß Peptide, die an einen bestimmten HLA-Subtyp binden, sich hinsichtlich ihrer nicht für die HLA-Bindung maßgeblichen Sequenz unterscheiden, indem sie z.B. von unterschiedlichen Proteinen desselben pathogenen Erregers oder von verschiedenen Erregern abgeleitet sind. Von einer solchen Variabilität kann eine Verstärkung der Stimulierung der Immunantwort bzw, eine Immunisierung gegen verschiedene Erreger erreicht werden.

Die Menge an wirksamem Peptid in der erfindungsgemäßen Zusammensetzung kann über einen breiten Bereich variieren. Die Menge an Peptid hängt u.a. von der Verabreichungsart und der jeweiligen Formulierung ab. Die zu verabreichende Menge an Peptid kann ca. 1.0 µg bis ca. 5000 µg pro Dosis betragen, im allgemeinen 1.0 µg bis ca. 1000 µg, insbesondere ca. 10 µg bis ca. 500 µg. Die Verabreichung kann ein- oder mehrmals erfolgen, bei mehrmaliger Verabreichung zweckmäßig mindestens dreimal. Insbesondere bei der therapeutischen Anwendung kann eine Applikation in Intervallen (z.B. von 1x pro Woche bis 1x pro Monat) über einen beliebig langen Zeitraum erfolgen, der durch den spezifischen Immunstatus des Patienten bzw. den Krankheitsverlauf bedingt ist.

Die erfindungsgemäße pharmazeutische Zusammensetzung kann auch *ex vivo* angewendet werden: Das Prinzip einer möglichen *ex vivo* Anwendung besteht darin, APCs, z.B. dendritische Zellen, *ex vivo* zu kultivieren, die Zellkultur mit der erfindungsgemäßen Zusammensetzung zu inkubieren und die APCs, die nun das Peptid im MHC-Kontext präsentieren, dem zu behandelnden Individuum zu verabreichen. Für diese Anwendungsmöglichkeit können literaturbekannte Methoden verwendet werden, wie z.B. von Porgador und Gilboa, 1995; Young und Inabe, 1996, beschrieben.

Das in der erfindungsgemäßen Zusammensetzung enthaltene Adjuvans hat die die Eigenschaft, den Eintritt des Peptids in die Zellen bzw. die Bindung des Peptides an die Zellen des Patienten zu erleichtern und die Immunogenität des Peptids zu verstärken. Das Adjuvans kann z.B. die Membranen von Zielzellen, in die das Peptid gelangen soll, zumindest kurzfristig durchlässig machen, um auf diese Weise das Peptid in die Zelle zu befördern. Dafür dürfte es von Vorteil, aber nicht unbedingt erforderlich sein, daß das Peptid an das Adjuvans gebunden wird, z.B. über elektrostatische Wechselwirkung zwischen elektronegativem Peptid und polykationischem Adjuvans. Es kann ein Import des Peptids in die Zelle auch dadurch bewirkt werden, daß das Peptid aufgrund seiner räumlichen Nähe zur Zellmembran durch diese hindurch gelangen kann, sobald das Adjuvans deren Durchlässigkeit bewirkt hat. Die Wirkung des Adjuvans kann auch darauf beruhen, daß es die für die Aufnahme in die Zelle kritische Konzentration des Peptids an der Zelloberfläche erhöht oder daß es die Phagozytose oder den Flüssigtransport (Pinozytose) des Peptids in die Zelle bewerkstelligt.

Überraschenderweise verstärkt die Gegenwart des erfindungsgemäßen Adjuvans nicht nur die Aufnahme des Peptids in die Zelle, sondern resultiert auch in einer Verstärkung der immunmodulatorischen Wirkung des Peptids, die auf eine korrekte Präsentation des Peptids durch MHC-Moleküle zurückzuführen sein dürfte.

Das erfindungsgemäße Adjuvans ist eine basische Polyaminosäure.

Der Polymerisationsgrad der Polyaminosäuren kann über einen weiten Bereich variieren. Er beträgt ca. 5 bis ca. 1000, insbesondere ca. 15 bis ca. 500.

Im Rahmen der vorliegenden Erfindung wird Polyarginin als Adjuvans verwendet.

Um den Mechanismus des durch basische Polyaminosäuren vermittelten Peptidtransports zu untersuchen, wurde im Rahmen der vorliegenden Erfindung die Freisetzung von Lactatdehydrogenase (LDH) gemessen. Während in Polyarginin-behandelten Proben die Konzentrationen an freigesetzter LDH praktisch nicht nachweisbar waren, wurden nach Inkubation mit Polylysin in den Zellüberständen hohe LDH-Konzentrationen nachgewiesen. Diese Ergebnisse lassen darauf schließen, daß die Wirkung von Polylysin auf eine Permeabilisierung der Zellmembran zurückzuführen sein dürfte.

Ohne auf die Theorie festgelegt sein zu wollen, dürfte die Wirkung der erfindungsgemäßen pharmazeutischen Zusammensetzung darin bestehen, daß das Peptid mit Hilfe des Adjuvans in die Zielzellen eindringt oder an Zellen bindet, die im endodermalen Bereich der Haut vorkommen. Zielzellen sind u.a. Antigen-präsentierende Zellen, von denen das Peptid, gegebenenfalls nach Prozessierung, den B- und/oder T-Zellen präsentiert wird. Beispiele für Zielzellen sind Makrophagen, Fibroblasten, Keratinozyten, Langerhanszellen, dendritische Zellen oder B-Zellen.

Im Rahmen der vorliegenden Erfindung wurde untersucht, ob kleine Peptide in Gegenwart von basischen Polyaminosäuren oder glykosylierten Formen von Polykation in verstärktem Ausmaß von Makrophagen-artigen Antigenpräsentierenden Zellen (APCs) aufgenommen werden. Von den verwendeten Zuckerresten ist bekannt, daß sie von Makrophagen über Rezeptor-vermittelte Endozytose aufgenommen werden. (Von APCs wird angenommen, daß sie *in vivo* den Zelltyp darstellen, der die Peptide aufnimmt und sie anderen Immunzellen präsentiert. Ergebnisse von *in vitro* Versuchen, die zeigen, daß APCs in Gegenwart der getesteten Adjuvantien erhöhte Mengen von Peptid-Antigenen endozytieren, sind ein Hinweis darauf, daß diese Adjuvantien auch *in vivo* geeignet sind, die Präsentation der Peptide gegenüber den zytotoxischen Effektorzellen sowie deren Aktivierung zu verstärken, was zu einer insgesamt verstärkten Immunantwort gegen das in der Vakzine enthaltene Target führt.)

Die Testung von Adjuvantien kann prinzipiell nach denselben Methoden erfolgen wie die Testung der Peptide, gegebenenfalls in mehreren Schritten:

Die Fähigkeit eines Adjuvans, die Bindung und/oder Internalisierung eines Peptids an APCs zu erhöhen, kann z.B. in einem ersten Schritt gemessen werden, indem APCs mit fluoreszenzmarkierten Peptiden und Adjuvans inkubiert werden. Eine durch das Adjuvans bewirkte erhöhte Aufnahme bzw. Bindung kann durch Vergleich mit Zellen, die mit Peptid allein versetzt wurden, mittels Durchflußzytometrie bestimmt werden.

In einem zweiten Schritt können die zu testenden Adjuvantien *in vitro* daraufhin untersucht werden, ob und in welchem Ausmaß ihre Gegenwart in einer Präsentation eines Peptids auf APCs resultiert, wobei nach den oben für die Testung der Peptide beschriebenen Methoden die MHC-Konzentration auf den Zellen gemessen werden kann.

Eine weitere Möglichkeit zur Testung der Effizienz eines Adjuvans ist die Verwendung eines *in vitro* Modellsystems. Hierbei werden APCs zusammen mit Adjuvans und Peptid inkubiert und die relative Aktivierung eines T-Zellklons, der das verwendete Peptid spezifisch erkennt, gemessen (Coligan et al.,1991; Lopez et al.,1993)

Die Effizienz der Formulierung kann auch über die zelluläre immunantwort durch den Nachweis einer "delayed-type hypersensitivity" (DTH)-Reaktion in immunisierten Tieren gezeigt werden.

Letztlich wird die immunmodulatorische Wirkung der Formulierung im Tierversuch gemessen. Hierzu können u.a. etablierte Tumormodelle, bei denen von Immunzellen erkannte Peptidsequenzen bekannt sind, eingesetzt werden. Die Vakzine, enthaltend Peptid und Adjuvans, wird in variierenden Verhältnissen, bezogen auf Peptid zu Adjuvans und Gesamtmenge, appliziert. Der Schutz vor Tumorwachstum ist ein Maß für die Wirksamkeit einer Tumorvakzine.

Die pharmazeutische Zusammensetzung kann parenteral, topisch, oral oder lokal verabreicht werden. Vorzugsweise wird sie parenteral, z.B. subkutan, intradermal oder intramuskulär verabreicht, vorzugsweise subkutan oder intradermal, um vor allem Hautzellen (Keratinozyten, Fibroblasten), dendritische Zellen, Langerhanszellen oder Makrophagen als Zielzellen zu erreichen. Im Rahmen einer Tumortherapie kann die Tumorvakzine auch intratumoral verabreicht werden.

Die erfindungsgemäße Zusammensetzung für die parenterale Verabreichung liegt im allgemeinen als Lösung oder Suspension des Peptids und des Adjuvans in einem pharmazeutisch annehmbaren Träger vor, vorzugsweise einem wässerigen Träger. Als wässerige Träger können z.B. Wasser, gepuffertes Wasser, Salzlösung (0.4 %) Glycinlösung (0.3 %), Hyaluronsäure und ähnliche bekannte Träger verwendet werden. Neben wässerigen Trägern können auch Lösungsmittel wie Dimethylsulfoxid, Propylenglycol; Dimethylformamid und Mischungen davon verwendet werden. Die Zusammensetzung kann außerdem pharmazeutisch annehmbare Hilfsstoffe enthalten, wie Puffersubstanzen sowie anorganische Salze, um einen normalen osmotischen Druck und/oder eine wirksame Lyophilisierung zu erreichen. Beispiele für derartige Zusätze sind Natrium- und Kaliumsalze, z.B. Chloride und Phosphate, Saccharose, Glukose, Proteinhydrolisate, Dextran, Polyvinylpyrrolidon oder Polyethylenglycol. Die Zusammensetzungen können mittels herkömmlicher Techniken sterilisiert werden, z.B. mittels Sterilfiltration. Die Zusammensetzung kann in dieser Form unmittelbar abgefüllt oder auch lyophilisiert und vor dem Gebrauch mit einer sterilen Lösung gemischt werden.

In einer Ausführungsform liegt die erfindungsgemäße pharmazeutische Zusammensetzung als topische Formulierung, z.B. zur dermalen bzw. transdermalen Applikation vor. Die pharmazeutische Zusammensetzung kann z.B als Hydrogel auf Polyacrylsäure- oder Polyacrylamid-Basis vorliegen (wie z.B. Dolobene®, Merckle), als Salbe, z.B. mit Polyäthylenglykol (PEG) als Vehikel, wie die Standardsalbe DAB 8 (50 % PEG 300, 50 % PEG 1500), oder als Emulsion, vor allem als Mikroemulsion auf Wasser-in-Öl- bzw. Öl-in-Wasser-Basis, gegebenenfalls auch mit Zusatz von Liposomen. Als Permeationsbeschleuniger ("Schlepper") eignen sich u.a. Sulfoxid-Derivate wie Dimethysulfoxid (DMSO) oder Decylmethysulfoxid (Decyl-MSO) sowie Transcutol (Diethylenglykolmonoethylether) oder Cyclodextrine, des weiteren Pyrrolidone, z.B. 2-Pyrrolidon, N-Methy-2-pyrrolidon, 2-Pyrrolidon-5-carbonsäure oder das biologisch-abbaubare N-(2-Hydroxyethyl)-2-pyrrolidon und deren Fettsäureester, Harnstoffderivate, wie Dodecylharnstoff, 1,3-Didodecylharnstoff und 1,3-Diphenylharnstoff, Terpene, z.B. D-Limonen, Menthon, a-Terpinol, Carvol, Limonenoxid, oder 1,8-Cineol.

Weitere Anwendungsformen sind Aerosole, z.B. zur Verabreichung als Nasenspray oder zur Inhalation.

Die erfindungsgemäße Zusammensetzung kann auch mittels Liposomen verabreicht werden, die in Form von Emulsionen, Schäumen, Micellen, unlöslichen Monolayers, Phospholipiddispersionen, Lamellarschichten und ähnlichem vorliegen können. Diese dienen als Vehikel, um die Peptide zielgerichtet zu einem bestimmten Gewebe, z.B. lymphoidem Gewebe oder Tumorgewebe, zu befördern bzw. die Halbwertszeit der Peptide zu verlängern.

Im Falle des Vorliegens der erfindungsgemäßen Zusammensetzung als topische Formulierung kann diese auch UV-Absorber enthalten, um z.B. bei der prophylaktischen Anwendung gegen Melanom gleichzeitig als Sonnenschutzmittel zu wirken.

Der Fachmann kann geeignete Hilfsstoffe und Formulierungen Standardwerken, wie "Remington's Pharmaceutical Sciences", 1990, entnehmen.

### Figurenübersicht

- Fig. 1:: Vakzinierung von DBA/2 Mäusen gegen Mastozytom P815 mit Peptid KYQAVTTTL
- Fig. 2:: Vakzinierung von DBA/2 Mäusen gegen Mastozytom P815 mit
- Fig. 3:: Peptid SYFPEITHI Vakzinierung von DBA2-Mäusen gegen Mastozytom P815 mit
- Fig. 4:: Peptid SYFPEITHI Vakzinierung von DBA/2-Mäusen gegen Melanom M-3 mit einer Mischung von Peptiden
- Fig. 5:: Vakzinierung von DBA/2-Mäusen gegen Melanom M-3 mittels topischer Applikation
- Fig. 6:: Vakzinierung von DBA/2 Mäusen gegen Melamom M-3-Metastasen
- Fig. 7:: Heilung von M-3 Mikrometastasen tragenden Mäusen nach Vakzinierung mit einer Peptidmischung
- Fig. 8:: Polylysin-vermittelte Beladung von Zellen mit Tyrosinase
- Fig. 9:: T-Zell-Aktivierung nach Vakzinierung im therapeutischen Modell (IFN-γ-Freisetzung von Milzzellen vakzinierter Tiere als Reaktion auf M-3-Zellen)
- Fig. 10:: Induktion antiviraler Immunität mittels lnfluenzanukleokapsid-Peptid ASNENMETM und fukosyliertem Polylysin als Adjuvans (CTL-Aktivierung)
- Fig. 11:: Verstärkung der Bindung von Peptiden an APCs durch basische Polyaminosäuren
- Fig. 12:: Permeabilisierung der Zellmembran durch basische Polyaminosäuren (LDH-Freisetzung nach Behandlung von Zellen mit Polylysin oder Polyarginin)
- Fig. 13:: Internalisierung der Peptide durch Polyarginin
- Fig. 14:: Transport von Peptiden in Antigen-präsentierende Zellen aus Knochenmark
- Fig. 15:: Steigerung des Transports von Peptiden in Antigen-präsentierende Zellen mittels polykationischen Polyaminosäuren und Histonen
- - Fig. 16:: Transporteffizienz in Abhängigkeit vom Polymerisationsgrad basischer Aminosäuren
- Fig. 17:: Transport von Peptiden mit Polyargininen niedrigen Molekulargewichts
- Fig. 18:: Beeinflussung der Transporteffizienz in Abhängigkeit der Ladung des Peptids

In den folgenden Beispielen wurden, wenn nicht anders angegeben, die folgenden Materialien und Methoden verwendet:

### A) Zellen

### a) Zellinien

Die Maus-Melanomzellinie Cloudman S91 (Klon M-3; ATCC No. CCL 53.1 ), die Mastozytomzellinie P815 (ATCC Nr. TIB 64) und die Monozyten-Makrophagen-Zellinie P388D1 (ATCC TIB 63) wurden von ATCC erworben. Die Zellen wurden in DMEM-Medium mit hohem Glukosegehalt ("High Glucose DMEM, Life Technologies), ergänzt mit 10 % FCS, 2 mM L-Glutamin und 20 µ/ml Gentamycin kultiviert. Die Zellen wurden routinemäßig auf Fehlen von Mycoplasmen-Kontamination getestet (PCR-Mycoplasma Detection Kit, Stratagene).

Die murine Zellinie RMA/S (Maus-Lymphom) wurde von Kärre et al, 1986 und von Ljunggren et al., 1990, beschrieben.

### b) Antigen-präsentierende Zellen aus Knochenmark

Zunächst wurden die Oberschenkelknochen von DBA/2-Mäusen gespült. Die Knochenmarkszellen wurden in DMEM-Medium mit hohem Glukosegehalt, enthaltend 10 % FCS, 5 % Pferdeserum, 2 mM L-Glutamin und 20 µg/ml Gentamycin in Gegenwart von 200 Einheiten/ml Maus-GM-CSF (Li et al., 1989; Genzyme, Cambridge, MA, USA) kultiviert. Während der ersten fünf Tage wurden alle 24 h zwei Drittel des Mediums ausgetauscht, um nichtadhärente Granutozyten und B-Zellen zu entfernen (Inaba et al., 1992). Sowohl adhärente als auch lose anhaftende Zellen wurden zwischen den Tagen 8 und 10 durch Inkubation mit PBS/5 mM EDTA geerntet und bei einer Zelldichte von 3 x10⁴ Zellen pro Weil auf 8-Well-Mikroskop-Objektträger (Nunc, Roskilde, Dänemark) ausgesät. Mehr als 90 % der Zellen zeigten eine positive Reaktion mit dem Antikörper F4/80 (Endogen, Cambridge, MA, USA).

### B) Peptidsynthese

Die Peptide wurden auf einem Peptid-Synthesizer (Modell 433 A mit Feedbackmonitor, Applied Biosystems, Foster City, Kanada) unter Verwendung von TentaGel S PHB (Rapp, Tübingen) als Festphase nach der Fmoc-Methode (HBTU-Aktivierung, Fastmoc^{TM}, Maßstab 0:25 mmol) synthetisiert. Die Peptide wurden in 1 M TEAA, pH 7.3 aufgelöst und mittels reverser Chromatographie auf einer Vydac C 18-Säule gereinigt. Die Sequenzen wurden mittels Flugzeitmassenspektrometrie auf einem MAT Lasermat (Finnigan, San Jose, Kanada) bestätigt.

### C) Liste der verwendeten Peptide

| Peptid Bezeichung | Sequenz | zugehöriges Antigen | Aminosäure Numerierung im Protein | MHC-Haplotyp |
|---|---|---|---|---|
| kpep117 | SYFPEITHI | Tyrosin-kinase JAK1 | 355-363 | H2-Kd |
| kpep118 | KYQAVTTTL | Tum-P198 | 14-22 | H2-Kd |
| Kpep162 | GPPHSNNF GY | Tum-P35B | 4-13 | H2-D^{d} |
| Kpep163 | ISTQNHRAL | P91A | 12-20 | H2-L^{d} |
| Kpep164 | LPYLGWLVF | P815 | 35-43 | H2-L^{d} |
| kpep143 | RYAEDYEEL | trp-1 | | H2-Kd |
| kpep145 | PYLEQASRI | tyrosinase | | H2-Kd |
| kpep146 | YYVSRDTLL | tyrosinase | | H2-Kd |
| kpep150 | YYSVKKTFL | trp-1 | | H2-K^{d} |
| | ASNENMETM | InfluenzanukleokapsidPeptid | | H2-K^{b} |

### Peptidmischungen:

Peptidmischung I für M-3 Melanomvakzine:
   kpep143, kpep145, kpep146, kpep150.
Peptidmischung III für Mastozytom P815 Vakzine:
   kpep117, kpep118, Kpep162, Kpep163, Kpep164

### D) Herstellung der Vakzinen

### D1) Einzelpeptidvakzinen

a) Einzelpeptidkontrollvakzinen ohne Adjuvans wurden herstellt, indem das Peptid in einer Konzentration von 1 mg/ml in PBS aufgenommen wurde. Die Inkubationszeit bis zur Injektion betrug 4 h bei Raumtemperatur.
b) Einzelpeptidvakzinen mit Polylysin (wenn im folgenden nicht anders angegeben, wurde Polylysin einer Kettenlänge von 200 verwendet) als Adjuvans wurden herstellt, indem Peptid und Polylysin in den angegebenen Mengen in HBS gemischt wurden. Die Inkubationszeit bis zur Injektion betrug 4 h bei Raumtemperatur.
i) Um eine Vakzine mit einem Gehalt von 16 µg wirksamen Peptids zu erhalten, wurden 11.8 µg Polylysin mit 160 µg Peptid kpep117 in einem Gesamtvolumen von 1 ml HBS gemischt.
ii) Um eine Vakzine mit einem Gehalt von 100 µg wirksamen Peptids zu erhalten, wurden 74 µg Polylysin mit 1 mg Peptid kpep117 in einem Gesamtvolumen von 1 ml HBS gemischt.
c) Einzelpeptidkontrollvakzinen mit Incomplete Freund's Adjuvans (IFA) wurden herstellt, indem Peptid und IFA in den angegebenen Mengen emulgiert wurden. Die Inkubationszeit bis zur Injektion betrug 30 min bei Raumtemperatur.
i) Für eine Kontrollvakzine, enthaltend 16 µg wirksames Peptid, wurden 192 µg Peptid kpep117 in 600 µl HBS mit 600 µl IFA emulgiert.
ii) Für eine Kontrollvakzine, enthaltend 100 µg wirksames Peptid, wurden 1.2 mg Peptid kpep117 in 600 µl HBS mit 600 µl IFA emulgiert.

### D2) Peptidmischungen als Vakzine

a) Peptidmischung I als Kontrollvakzine ohne Adjuvans enthielt 250 µg jedes der Peptide kpep143, kpep145, kpep146, kpep150 in einem Gesamtvolumen von 1 ml PBS.
b) Peptidmischung III als Kontrollvakzine ohne Adjuvans enthielt 250 µg jedes der Peptide kpep117, kpep118, Kpep162, Kpep163, Kpep164 in einem Gesamtvolumen von 1 ml PBS.
c) Peptidmischung 1 als Vakzine mit Polylysin als Adjuvans wurde herstellt, indem 1 mg Peptidmischung I (enthaltend 250 µg jedes Peptides) mit 74 µg Polylysin in HBS gemischt wurden. Die Inkubationszeit bis zur Injektion betrug 4 h bei Raumtemperatur.
d) Peptidmischung III als Vakzine mit Polylysin als Adjuvans wurde herstellt, indem 1.25 mg Peptidmischung III (enthaltend 250 µg jedes Peptides) mit 93 µg Polylysin in HBS gemischt wurden. Die Inkubationszeit bis zur Injektion betrug 4 h bei Raumtemperatur.
e) Peptidmischung I als Kontrollvakzine mit Incomplete Freund's Adjuvans wurde herstellt, indem 1.2 mg Peptidmischung I in 600 µl HBS (enthaltend 300 µg jedes Peptides) mit 600 µl IFA emulgiert wurden. Die Inkubationszeit bis zur Injektion betrug 30 min bei Raumtemperatur.
f) Peptidmischung III als Kontrollvakzine mit Incomplete Freund's Adjuvans wurde herstellt, indem 1.5 mg Peptidmischung III in 600 µl HBS (enthaltend 300 µg jedes Peptids) mit 600 µl IFA emulgiert wurden. Die Inkubationszeit bis zur Injektion betrug 30 min bei bei Raumtemperatur.
g) Für die topische Verabreichung mit Polylysin als Adjuvans wurden 1 mg Peptidmischung I (enthaltend 250 µg jedes Peptids) mit 74 µg Polylysin 4 h lang in einem Gesamtvolumen von 400 µl HBS inkubiert. Die erhaltene Mischung wurde in 1.6 g des Hydrogels DOLOBENE (Merckle) eingerührt.
h) Für die topische Verabreichung einer Kontrollvakzine ohne Adjuvans wurde 1 mg Peptidmischung I (enthaltend 250 µg jedes Peptids) in einem Gesamtvolumen von 200 µl HBS in 1.8 g des Hydrogels DOLOBENE (Merckle) eingerührt.
i) Die Herstellung von Fukose-gekoppeltem Polylysin (Kettenlänge: 240 bzw. 200) wurde nach der von MacBroom et al., 1992, beschriebenen Methode vorgenommen, wobei eine Substituierung von ca. 40% erzielt wurde (die Ausgangsmaterialien β-L-Fucopyranosylphenyl-isothiocyanat und Polylysin wurden von Sigma bezogen).
j) Wenn Transferrin/Polylysin-Konjugate (hergestellt, wie in der WO 93/07283 beschrieben) verwendet wurden, wurde die Menge so eingestellt, daß die absolute Menge an Polylysin 75 µg pro mg Peptid betrug. Wenn Plasmid-DNA (Leerplasmid pSP65, LPS-frei, Boehringer Mannheim) in die Komplexe integriert wurde, betrug das Verhältnis 37.5 µg DNA/75 µg Polylysin/1 mg Peptid. Im Falle der Verwendung von 160 µg statt 1 mg Peptid wurden die Mengen der anderen Komponenten um denselben Faktor (6.25) reduziert.

### E) Injektion der Vakzine

Vor der subkutanen Injektion wurden die Mäuse in Gruppen von bis zu acht Tieren in einer isolierten Luftkammer anästhesiert. Nach 3.5 min Halothan-Behandlung (4 % in O₂, Flußrate 4) waren die Mäuse ca. 1 min lang betäubt; in dieser Zeit wurde die Vakzine subkutan injiziert.

Die intraperitoneale Injektion erfolgte ohne vorherige Anästhesierung. Das Injektionsvolumen war für jede Vakzine 100 µl pro Tier, das entspricht 100 µg Einzelpeptid oder Peptidmischung I pro Tier. Im Fall von Peptidmischung III wurden 125 µg Gesamtpeptidmenge pro Maus appliziert.

### F) Topische Anwendung der Vakzine

Pro Maus wurden 200 mg Salbe, enthaltend 100 µg Peptid oder Peptidmischung I bzw. 125 µg Peptidmischung I, in die Haut der rasierten Tiere eingerieben, und zwar in den gesamten Rücken und in die Ohren. Die richtige Menge wurde mit einer Waage kontrolliert.

### G) Anwendung der Vakzine gegen Tumorwachstum im Mausmodell

Das Protokoll der Testung der Wirksamkeit der Krebsvakzinen im prophylaktischen oder im therapeutischen Mausmodell entsprach, wenn nicht anders angegeben, dem in der WO 94/21808 beschriebenen Prinzip, wobei als Mausmodell das DBA/2-Modell verwendet wurde.

### Beispiel 1

### Vakzinierung von DBA/2 Mäusen gegen Mastozytom P815

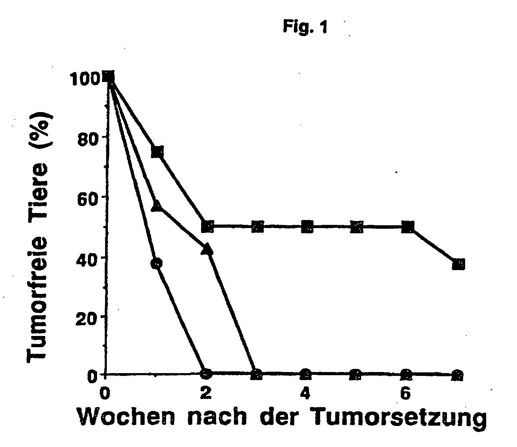

160 µg des Peptids der Sequenz KYQAVTTTL (kpep118), abgeleitet von dem von Lethe et al., 1992, beschriebenen Tumorantigen P815, einem Liganden von H2-K^{d}, wurden mit 11.8 µg Polylysin300 in 500 µl HBS gemischt und 4 h bei Raumtemperatur inkubiert. Dann wurden 500 µl EBSS (Earl's gepufferte Salzlösung) beigegeben. Je 100 µl der erhaltenen Mischung wurden 8 Mäusen in einwöchigem Abstand subkutan verabreicht. Nach dieser Vorimmunisierung wurden nach einer weiteren Woche Tumoren gesetzt, indem jeder Maus contralateral 5 x 10⁴ Zellen der Mastozytomzellinie P815 (ATCC Nr. TIB 64; diese Zellen exprimieren das Tumorantigen, von dem das Peptid P815 abgeleitet ist) in 100 µl EBSS injiziert wurden. Das Ergebnis dieser Versuche ist in Fig. 1 (gefüllte Quadrate) dargestellt .

In einem Parallelversuch wurden 200 µg des Peptids mit 500 µl HBS gemischt und anschließend mit 500 µl Freund's Adjuvans emulgiert. Mit je 100 µl der erhaltenen Emulsion wurden 8 Mäuse vorimmunisiert und anschließend mit P815-Zellen Tumoren gesetzt, wie oben angegeben (Fig. 1: gefüllte Kreise).

Für einen weiteren Parallelversuch wurde eine zelluläre Tumorvakzine wie folgt hergestellt:

160 µg Peptid kpep 118 wurden mit 3 µg Transferrin-Polylysin (TfpL), 10 µg pL und 6 µg Plasmid psp65 (LPS-frei) in 500 µl HBS-Puffer gemischt. Nach 30 min bei Raumtemperatur wurde die obige Lösung in eine T 75 Zellkulturflasche mit 1.5 x 10⁶ Zellen der allogenen Fibroblastenzellinie NIH3T3 (ATCC Nr. CRL 1658) in 20 ml DMEM-Medium (10 % FCS, 20 mM Glukose) gegeben und bei 37°C inkubiert. Nach 3 h wurden die Zellen mit 15 ml frischem Medium versetzt und über Nacht bei 37°C und 5 % CO₂ inkubiert. 4 h vor der Applikation wurden die Zellen mit 20 Gy bestrahlt. Die Aufarbeitung der Vakzine erfolgte, wie in der WO 94/21808 beschrieben. Die Vorimmunisierung mit dieser Vakzine wurde in einwöchigem Abstand mit 10⁵ Zellen vorgenommen; nach einer weiteren Woche wurde die Tumorsetzung durchgeführt, wie oben beschrieben (Fig. 1: gefüllte Dreiecke). Es zeigte sich, daß die Vakzine, die das Peptid mit Polylysin vereinigt enthielt, die Mäuse am besten vor Tumorbildung schützte.

### Beispiel 2

### Vakzinierung von DBA/2 Mäusen gegen Mastozytom P815 mit einer Einzelpeptidvakzine

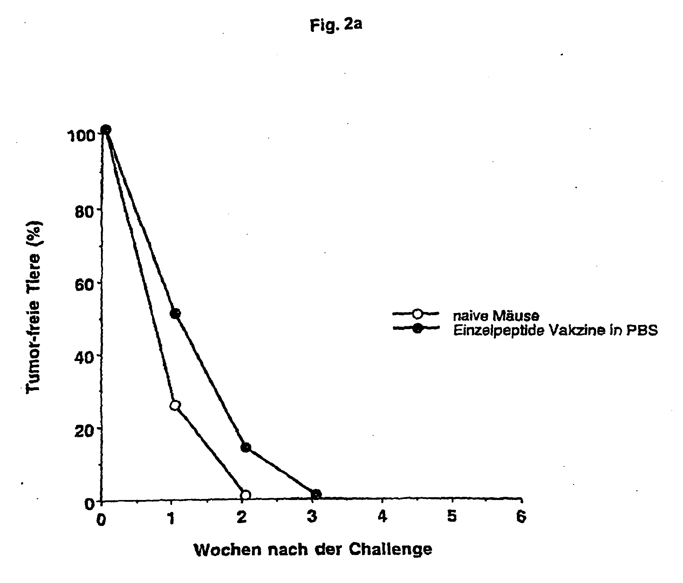

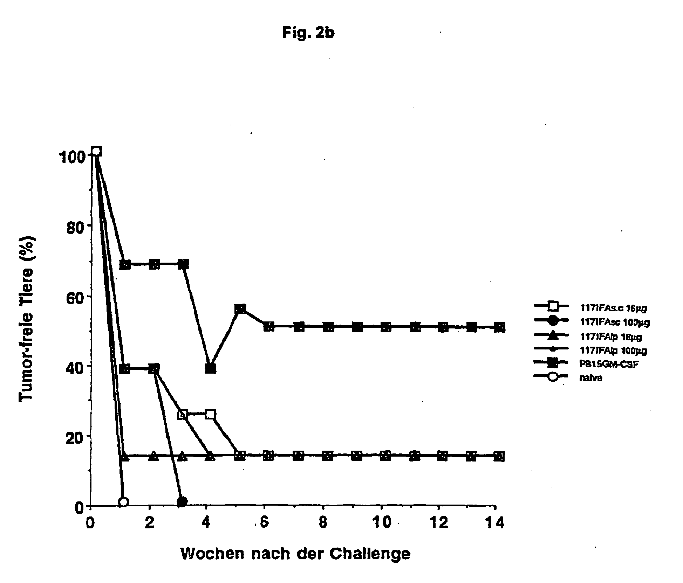

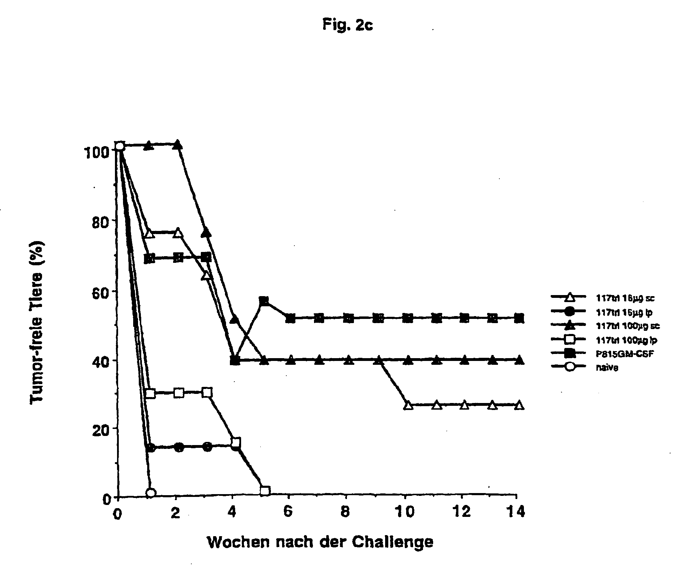

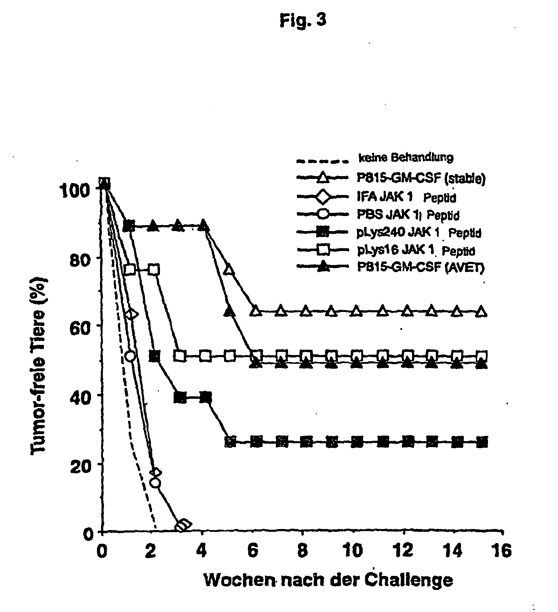

a) Drei Einzelpeptid-Vakzinen, enthaltend entweder Peptid kpep117 allein in PBS (Fig. 2a), Peptid kpep117, emulgiert in IFA (Fig. 2b), oder Peptid kpep117 mit Polylysin (Kettenlänge: 240) als Adjuvans (Fig. 2c), wurden auf ihre protektive Wirkung gegen eine P815-Tumorsetzung getestet. Die Vakzinen wurden, wie oben in Abschnitt D beschrieben, hergestellt. Das Injektionsvolumen betrug jedesmal 100 µl; die Injektion wurde subkutan (sc) oder intraperitoneal (ip) vorgenommen. Naive Mäuse dienten als Negativkontrolle, eine Ganzzellvakzine, bestehend aus GM-CSF sekretierenden P815 Zellen als Positivkontrolle (P815-GM-CSF; 10⁵ Zellen in 100 µl wurden pro Tier subkutan injiziert). Jede Versuchsgruppe bestand aus acht Tieren, es wurden drei Vakzinierungen (sc) in siebentägigen Abständen vorgenommen. Eine Woche nach der letzten Vakzinierung erhielten die Tiere eine contralaterale Tumorchallenge mit 5 x 10⁴ P815-Zellen. Die Tiere wurden täglich inspiziert, das Auftreten von Tumoren wurde in wöchentlichem Abstand kontrolliert.
   Das Peptid kpep117 mit Polylysin als Adjuvans erzielte die beste Anti-Tumorwirkung, wenn 100 µg pro Tier subkutan injiziert wurden (drei von acht Tieren geschützt). Dieser Effekt war annähernd so gut wie der mit der Ganzzellvakzine erzielte (vier von acht Tieren geschützt). 16 µg Peptid zusammen mit Polylysin pro Tier war weniger wirksam (zwei Tiere geschützt), aber deutlich besser als 100 µg Peptid in PBS (Fig. 2a, keine Schutzwirkung). Auch emulgiert in IFA erzielte das Peptid nicht die Wirkung, die es zusammen mit Polylysin leistet (Fig. 2c).
b) In einem weiteren Experiment im P815 Mastozytom-Modell wurden zwei unmodifizierte Polylysine unterschiedlicher Kettenlänge miteinander verglichen, ein kurzes mit nur 16 Lysinresten (pL16) und ein langes mit 240 Resten (pL240). Den Tieren der Kontrollgruppen wurden 100 µg Peptid kpep 117, entweder in PBS gelöst oder in IFA emulgiert, injiziert. Zwei GM-CSF sekretierende zellulläre Kontrollvakzine wurden als Positivkontrolle verwendet (vgl. a)), wobei eine Vakzine aus stabil transfizierten P815-Zellen und die zweite Vakzine mittels transienter Transfektion unter Verwendung der von Wagner et al., 1992, beschriebenen Methode (AVET) erhalten wurde. Die beiden Ganzzellvakzinen verliehen vier bzw. fünf von insgesamt acht Tieren Schutz. Die Vakzinen auf der Grundlage von Peptiden, die aus Peptid allein oder dem in IFA emulgierten Peptid bestanden, zeigten keine Schutzwirkung; alle Tiere entwickelten bald nach der Tumorsetzung Tumore. . Wenn jedoch das Peptid gemeinsam mit Polylysin verabreicht wurde, schützte die Peptidvakzine die Tiere gegen die Tumorsetzung: zwei von acht Tieren waren geschützt, wenn das lange Polylysin (pL240) verwendet wurde, und vier von acht Tieren im Fall des kurzen Polylysins. Diese Ergebnisse, die in Fig. 3 dargestellt sind, zeigen, daß ein einzelnes Peptid, wenn es mit Polylysin als Adjuvans verabreicht wird, einen effizienten Antitumorschutz bewirkt, der vergleichbar ist mit dem einer der wirkungsvollsten Zytokinsekretierenden Ganzzellvakzine, die in der Literatur als Standard für Antitumor-Vakzinierung herangezogen wird (Dranoff et al., 1993; Schmidt et al., 1995).

### Beispiel 3

### Vakzinierung von DBA/2 Mäusen gegen Mastozytom P815 mit einer Tumorvakzine, enthaltend P815-Einzelpeptide oder Mischungen von P815-Peptiden

Für die Herstellung der Vakzine wurden die folgenden Peptide verwendet:
kpep118 (100 µg pro Injektion)
Peptidmischung III (kpep117, kpep118, kpep162, kpep163, kpep164) diese Peptidmischung enthielt alle bisher bekannten P815-Peptide; pro Injektion wurden 25 µg jedes Peptids verabreicht).

Als positive Kontrolle wurden GM-CSF sekretierende P815-Zellen verwendet.

In Vorversuchen hatte sich kpep117 als das Peptid mit der besten Schutzwirkung gegen eine P815-Tumorsetzung erwiesen, wenn 100 µg Peptid zusammen mit Polylysin (7.5 µg Polylysin/100 µg Peptid, entsprechend dem Standardverhältnis; Polylysin: Kettenlänge = 200) verwendet wurden. Eine geringere Menge (16 µg) kpep117 war weniger wirksam gewesen. In diesem Beispiel wurden 100 µg kpep118 pro Tier injiziert, und zwar einmal nur mit Polylysin (Gruppe B), einmal mit Transferrin-Polylysin (Gruppe C) und einmal mit Transferrin-Polylysin/DNA (Gruppe D). Als Kontrolle wurde kpep118 mit IFA verwendet. In diesem Experiment zeigte kpep118 allein keine Schutzwirkung gegen die Tumorsetzung.

In den in Beispiel 4 durchgeführten Versuchen wurde gezeigt, däß eine Vakzine, enthaltend eine Peptidmischung aus Melanompeptiden, eine Schutzwirkung gegen Melanom aufweist. Es wurde daher in diesem Beispiel getestet, ob sich das Konzept der Peptidmischung auch für P815 eignet.

Die Peptidmischung III wurde einmal nur mit Polylysin (Gruppe E), einmal mit Transferrin-Polylysin (Gruppe F) und einmal mit Transferrin-Polylysin/DNA (Gruppe G) verabreicht. Als Kontrolle wurde Peptidmischung III in IFA verwendet. Als Negativkontrolle wurden naive Mäuse verwendet; als positive Kontrolle GM-CSF-transfizierte P815-Zellen (10⁵ Zellen pro Maus).

Die in diesem Beispiel durchgeführten Versuche entwickelten sich, im Vergleich zu den anderen Versuchen, eher untypisch: in der positiven Kontrollgruppe (GM-CSF sekretierende Zellen) entwickelten alle Tiere bald nach der Tumorsetzung Tumoren, wobei die meisten dieser Tumoren ebenso schnell verschwanden, wie sie entstanden waren. Eine mögliche Erklärung dafür ist, daß der Tumor eine Weile wuchs, bevor er zerstört wurde. Eine zweite mögliche Erklärung wäre, daß die als Tumor diagnostizierte Schwellung nicht vom Tumorwachstum herrührte, sondern von einem starken Immunzellinfiltrat (Granulom). Da die Tiere nicht seziert wurden, konnte die Ursache nicht definitiv festgestellt werden; jedenfalls wurden die gesetzten Tumoren letztlich zerstört. Ein weiteres interessantes Ergebnis wurde in Gruppe G erhalten, in der die Tiere mit einer Kombination aus Peptidmischung III und Polylysin behandelt wurden. Alle Tiere entwickelten Tumoren, aber in zwei der Tiere war die Größe der Schwellung des Tumors (bzw. des Immunzellinfiltrats) relativ gering, nahm nicht zu, und die Mäuse sahen nicht ungesund aus. Diese beiden Tiere wurden nicht getötet, sondern weiter unter Beobachtung gehalten. Überraschenderweise waren die Tumoren neun Wochen nach der Tumorsetzung nicht mehr nachweisbar, ein bisher nicht beobachtetes Ergebnis. Zwei von acht Tieren zerstörten schließlich ihre Tumorlast. Die Zerstörung der Tumoren dürfte auf den Gehalt an kpep117 in der Peptidmischung zurückzuführen sein, was in Analogie zu Beispiel 2 stünde, wo zwei von acht Tieren mit 16 µg kpep117 und drei von acht Tieren mit 100 µg kpep117 geschützt waren. Die Schutzwirkung könnte jedoch auch durch mehr als ein Peptid der Mischung hervorgerufen worden sein.

### Beispiel 4

### Schutz von DBA/2-Mäusen gegen Melanom M-3 durch Vorimmunisierung mit einer Tumorvakzine, enthaltend eine Mischung von Peptiden

Es wurde eine prophylaktische Vakzine verwendet, die eine Mischung von Melanompeptiden (Peptidmischung 1, Abschnitt D2) enthielt.

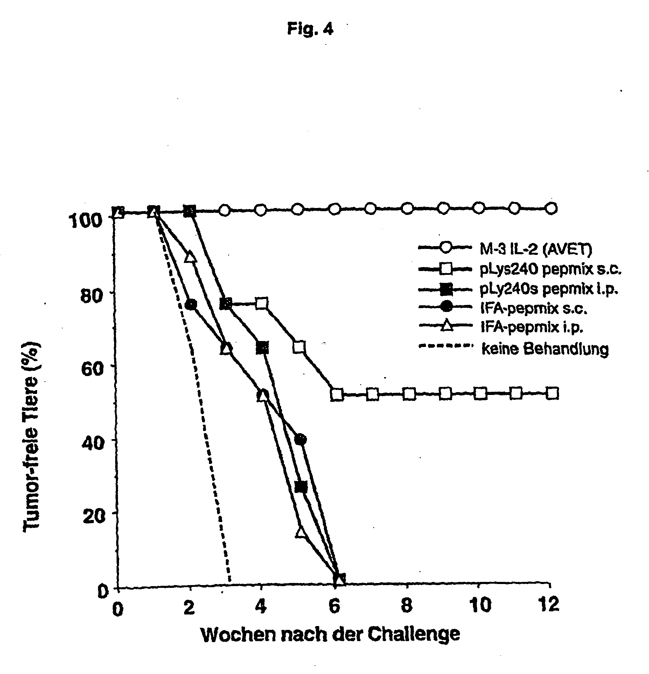

Das Protokoll der Vorimmunisierung mit der Vakzine sowie die Setzung der Tumoren entsprach dem in Beispiel 2 beschriebenen Protokoll, mit dem Unterschied, daß die Tumorsetzung mit M3-Zellen (10⁵ Zellen pro Tier) vorgenommen wurde. Als Kontrollvakzine wurde eine Ganzzellvakzine aus M-3-Zellen benutzt, die eine optimale Menge IL-2 (1000 - 2000 Einheiten pro 10⁵ Zellen) sekretiert und hergestellt wurde, wie von Schmidt et al., 1995, beschrieben. Unter den gewählten Versuchsbedingungen erzielte diese Vakzine einen 100^{%}igen Schutz (Fig. 4). Vier Gruppen von Versuchstieren wurden die Vakzinen mit der Peptidmischung verabreicht. Zwei Gruppen erhielten, entweder s.c. oder i.p., die Peptide emulgiert in IFA. Die anderen beiden Gruppen erhielten, entweder s.c. oder i.p., die Peptide zusammen mit Polylysin (pL240).

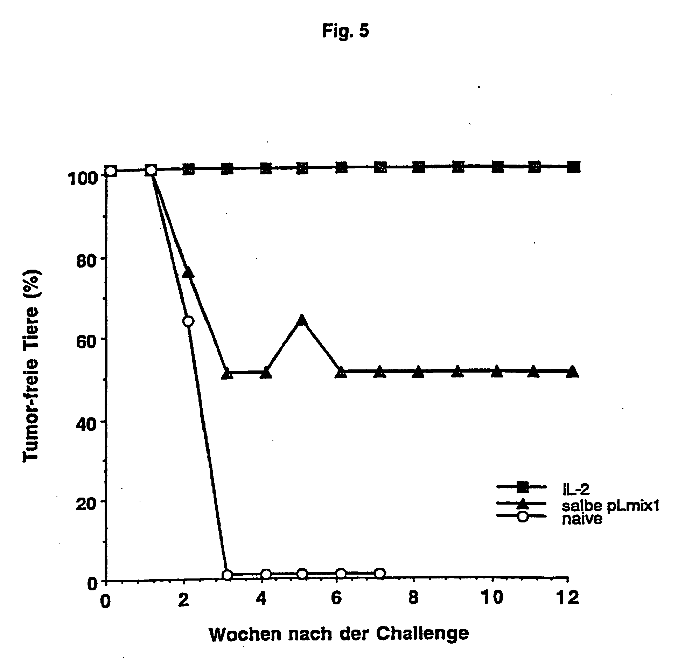

Fig. 4 illustriert den Schutzeffekt der Peptidvakzine mit Polylysin (pL240) als Adjuvans; 50 % der behandelten Mäuse waren gegen die M-3 Tumorchallenge geschützt im Vergleich zu unbehandelten Tieren, in denen sich rasch solide Tumoren entwickelten. Diese Wirkung konnte erzielt werden, wenn die Peptid-Polylysin-Vakzine subkutan injiziert oder als Hydrogel auf die Haut aufgetragen wurde (Fig. 5). In den anderen drei Kontrollgruppen, die mit den Peptid/Polylysin-Vakzinen i.p. oder mit Peptiden in IFA behandelt wurden, war die Vakzine im wesentlichen unwirksam. Hier wurden die gesetzten Tumoren nicht abgestoßen, und die Tumoren wuchsen im Vergleich zu den unbehandelten Kontrolltieren mit einer nur geringen Verzögerung. Diese Ergebnisse zeigen, daß die eine Peptidmischung enthaltende Vakzine eine Antitumor-Schutzwirkung erzielt, wenn sie Polylysin enthält. Unter den gewählten Versuchsbedingungen war diese Peptid-Vakzine nur halb so wirksam wie die zelluläre IL-2-Vakzine, welche, in Übereinstimmung mit kürzlich erschienenen Berichten (Zatloukal, 1993, Zatloukal, 1995), bis zu 100 % der Tiere gegen die Tumorsetzung mit 10⁵ lebenden M3-Zellen schützte.

### Beispiel 5

### Schutz von DBA/2 Mäusen gegen M-3 Metastasen

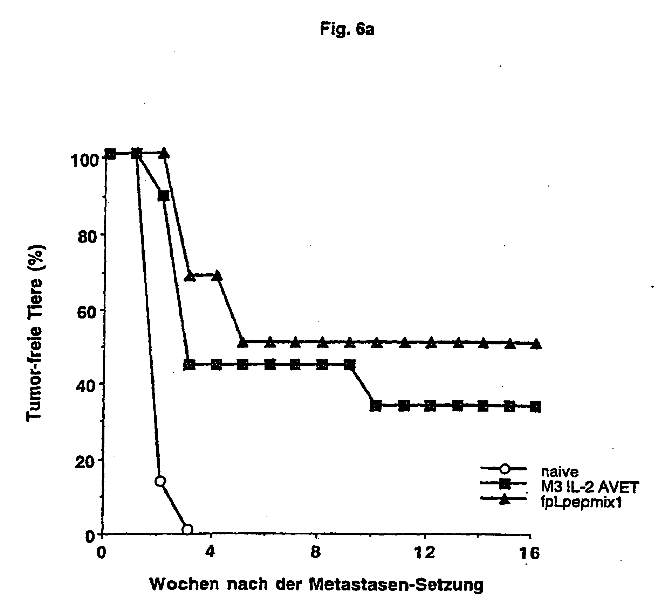

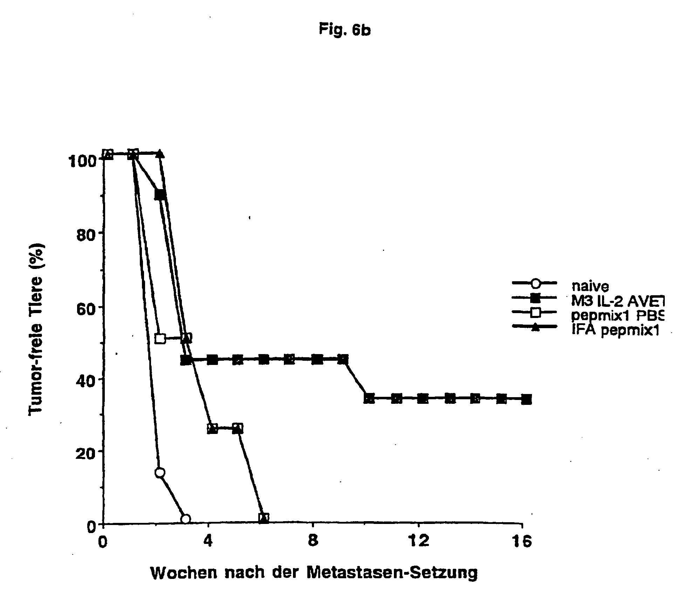

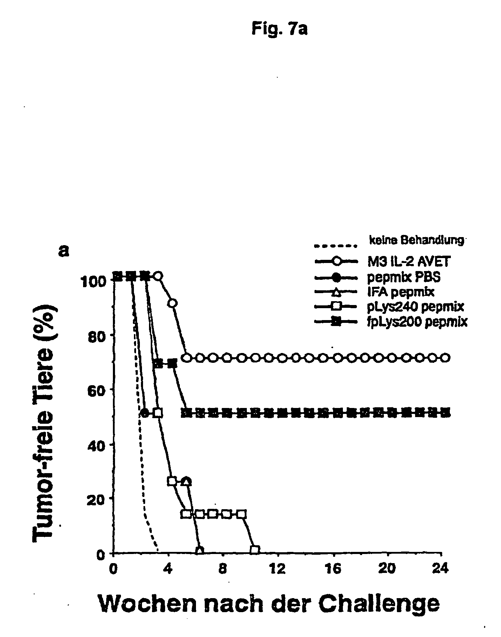

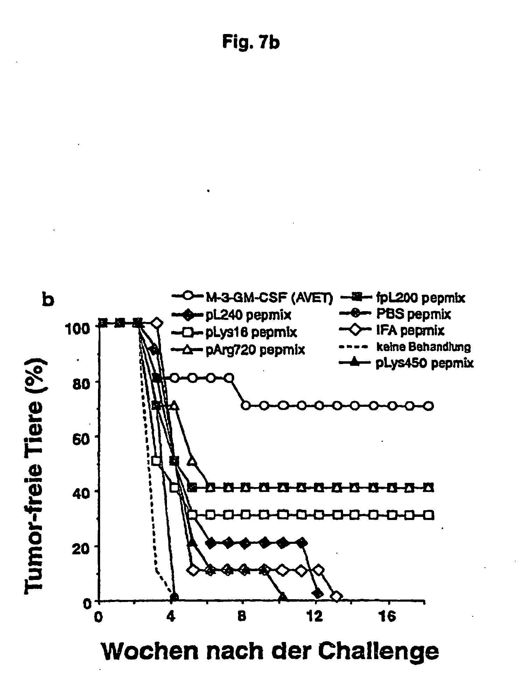

a) Es wurde eine therapeutische Vakzine verwendet, die eine Mischung von Melanompeptiden (Peptidmischung I, beschrieben in Abschnitt D2) enthielt. Es wurden drei Vakzinierungen (sc) in einwöchigem Abstand, verabreicht. Die erste Vakzinierung erfolgte fünf Tage nach der Metastasensetzung, es wurde demnach gegen eine Fünftagesmetastase geimpft. 1.2 x 10⁴ M-3 Zellen wurden für die Metastasensetzung injiziert, es wurde das in der WO 94/21808 und bei Schmidt et al., 1996, beschriebene Protokoll verwendet.
   Als Vakzine wurde Peptidmischung 1 verwendet: ohne Adjuvans (pepmix1 PBS), mit IFA als Adjuvans (IFA pepmix1) oder mit Fukose-modifiziertem Polylysin (fpL pepmix1). Kontrollgruppen erhielten keine Vakzine (naive) oder die in Beispiel 4 aufgeführten IL-2 produzierende M-3 Ganzzellenvakzine. Fig. 6 zeigt, daß der beste Schutz in der Gruppe erzielt wurde, die mit Peptidmischung I mit Fukose-modifiziertem Polylysin als Adjuvans behandelt wurde (Fig. 6a). 50 % der Mäuse konnten die Metastasen abstoßen (4/8). Diese Behandlung war sogar wirksamer als die mit der Ganzzellvakzine, die nur 33 % der Mäuse schützte (3/9). Die Peptidvakzine mit IFA oder ohne Adjuvans führte lediglich zu einer Verzögerung beim Auswachsen der Metastasen zum Tumor (Fig. 6b).
b) In einem weiteren Experiment wurde im therapeutischen Modell die Schutzwirkung einer Vakzine untersucht, die die Peptidmischung I enthielt und subkutan verabreicht wurde. Kontrollgruppen erhielten die Peptidmischung in PBS (ohne Adjuvans) oder mit IFA. Als Adjuvans wurde unmodifiziertes Polylysin 240 verwendet. Außerdem wurde als, Adjuvans Fukose-modifiziertes Polylysin 200 (fpL 200) verwendet. Als Kontrolle wurde eine Gruppe von Tieren mit der IL-2 exprimierenden zellulären Vakzine in dieses Experiment aufgenommen. Wie in Fig. 7a gezeigt wird, war die Peptid/Polylysin-Vakzine bei der Behandlung von M3-Metastasen wirksam. Eine signifikante Heilungsrate wurde in diesem Versuch nur mit dem Fukose-modifizierten Polylysin erreicht. Bei dieser Behandlung stießen 50 % der Tiere die Metastasen ab, was im Vergleich zur IL-2-Vakzine, die in diesem Fall 70 % der Tiere heilte, ein guter Wert ist.
c) In einem weiteren Experiment wurde im therapeutischen Modell getestet, wie sich Änderungen und Modifikationen des Polykations auf die Antitumorwirkung auswirken. Dabei wurden zusätzlich zum nichtmodifizierten und zum Fukose-modifizierten Polylysin 200 das kurze, nichtmodifizierte Polylysin pL16, ein langes Polylysin pL450 und ein weiteres Polykation, nämlich Polyarginin (pArg720) getestet. Als positive Kontrolle wurde eine zelluläre, eine optimale Menge (≥ 10 ng pro 10⁵ Zellen) GM-CSF sekretierende M3-Vakzine verwendet (Schmidt, 1995). Auch in diesem Versuch enthielten Kontrollgruppen die Peptidmischung in IFA oder ohne jedes Adjuvans. Wie auch im Beispiel 5 b) wurde die beste Wirkung erzielt, wenn Fukose-Polylysin als Adjuvans verwendet wurde Fig. 7b. In diesen Gruppen stießen 40 % der Tiere die Metastasen ab, im Vergleich zu 30 % in der Gruppe mit dem kurzen Polylysin pL16. Außer in der Gruppe, die die Peptide zusammen mit Polyarginin erhalten hatten, zeigten die Tiere der anderen Gruppen, denen eine Peptid-Vakzine verabreicht worden war, nur eine kurze Verzögerung bei der Entstehung von Tumoren. Das nichtmodifizierte Polyarginin war in diesem Versuch genauso wirksam wie das Fukose-modifizierte Polylysin, es führte zur Abstoßung der Metastasen in vier von zehn Tieren.

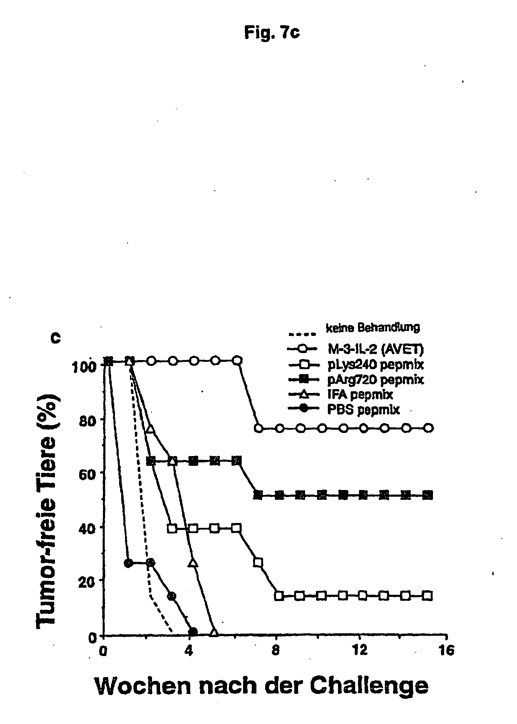

Fig. 7c zeigt die Wiederholung dieses durch Polyarginin erzielten Effekts in einem unabhängigen Experiment. Auch hier zeigte die Vakzinierung mit der Peptidmischung in Verbindung mit Polyarginin in vier von acht behandelten Tieren eine Antitumorwirkung.

### Beispiel 6

### Transloading von Zellen mit Tyrosinase unter Verwendung von Polylysin als Adjuvans

Dieses Beispiel diente als Versuch, der zeigen sollte, daß Polylysin als Adjuvans für die Beladung von Zellen mit größeren Proteinfragmenten oder ganzen Proteinen geeignet ist, wobei als Zellen stellvertretend M-3-Zellen verwendet wurden.

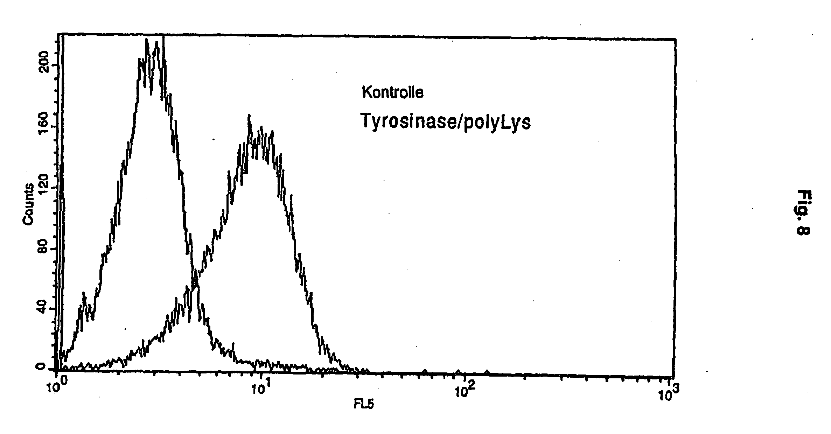

Für die Beladung der Zellen wurden zunächst 160 µg FITC-markierte Tyrosinase (EC 1.14.18.1; Sigma) mit 3 µg Polylysin (pL240) versetzt und für 3 h bei Raumtemperatur inkubiert. Dannach wurde die erhaltene Lösung in eine T 75 Zellkulturflasche mit 2 x 10⁶ M-3-Zellen gegeben und bei 37°C inkubiert. Danach wurden die Zellen zweimal mit PBS gewaschen, mit PBS/2 mM EDTA abelöst und in 1 ml PBS/5 % FCS zur FACS-Analyse aufgenommen. Fig. 8 zeigt die Beladung von M-3-Zellen mit Tyrosinase (die linke Kurve zeigt die Kontrolle, die rechte die Beladung mit Tyrosinase).

### Beispiel 7

### Bestimmung der T-Zell-Aktivierung nach Immunisierung im therapeutischen Modell

Nachdem die Peptid/Polykation-Vakzine im therapeutischen Mausmodell (vgl. Beispiel 5) eindeutig eine Wirkung gezeigt hatte, wurde untersucht, ob diese Behandlung auch zur Aktivierung von T-Zellen führt. Dafür wurde Zytokinsekretion nach Co-Inkubation von Milzzellen vakzinierter Tiere mit parentalen M3-Zellen stellvertretend als Marker herangezogen (Kawakami et al., 1994b).

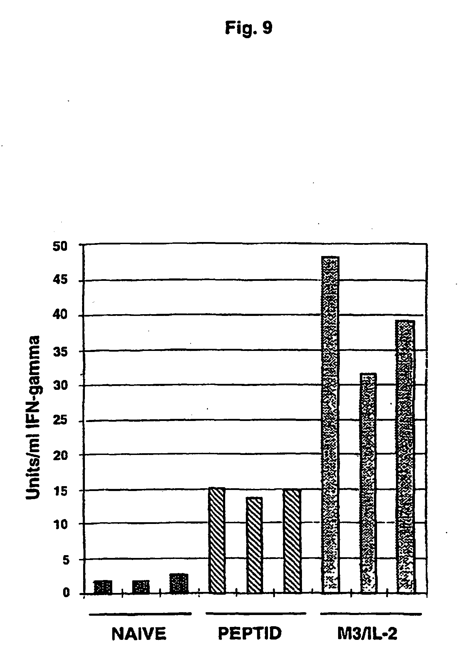

Es wurden Einzelzell-Milz-Suspensionen aus vakzinierten und unbehandelten Tieren hergestellt, gefolgt von Erythrozytenlyse mit hypotonischem Puffer (0.15 NH₄Cl, 1 mM KHCO₃, 0.1 mM EDTA, pH 7.4). Adhärente Zellen wurden entfernt, indem 3 x 10⁶ Milzzellen pro ml DMEM-Medium (10 % FCS) in Petrischalen 90 min lang bei 37°C inkubiert wurden. Nicht-adhärente Zellen wurden durch vorsichtiges Pipettieren und Co-Kultivieren mit 1 x 10³ parentalen Zellen in verschiedenen Mengenverhältnissen co-kultiviert. Die Zellen wurden in 200 µl DMEM-Medium (10 % FCS), 2 mM L-Glutamin und 20 µg/ml Gentamycin in 96 Well-Flachboden-Gewebekultuschalen kultiviert. Am Tag 9 wurden 100 µl Überstand geerntet und der jeweilige IFN-γ-Gehalt unter Verwendung eines kommerziell erhältlichen ELISA-Kits (Endogen, Cambridge, MA, USA) nach Vorschrift des Herstellers gemessen. Es zeigte sich, daß nach neun Tagen Inkubation nur Milzzellen von vakzinierten Tieren größere Mengen an IFN-γ ins Medium sekretierten, während in den Co-Kulturen von Milzzellen unbehandelter Tiere und M3-Zellen praktisch kein IFN-γ nachweisbar war. Das Ergebnis dieser Versuche ist in Fig. 9 dargestellt.

### Beispiel 8

### Induktion antiviraler Immunität mittels Influenzanukleokapsid-Peptid ASNENMETM und fukosyliertem Polylysin als Adjuvans

Es wurde eine Vakzine eingesetzt, die pro 1 mg Peptid ASNENMETM 75 µg fpLys enthielt. Die Vakzine wurde durch einmalige Injektion, wie im Methodenteil angegeben, verabreicht, wobei pro Tier 100 µg Peptid/7.5 µg fpLys injiziert wurden. Zur Kontrolle erfolgte die Injektion von 100 µg Peptid allein (PBS) beziehungsweise wurde keinerlei Injektion vorgenommen (naive Kontrolle).

RMA-S Maus-Lymphom-Zellen wurden über Nacht in serumfreiem Medium bei 26°C mit 10 µg/ml Peptid ASNENMETM inkubiert.

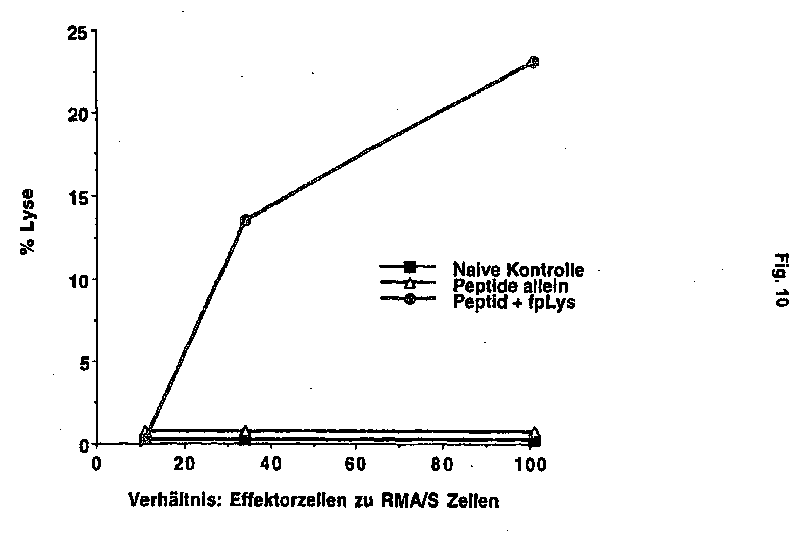

10 Tage nach der Vakzinierung wurden Milzzellen aus den vakzinierten Tieren isoliert, mit den Peptid-beladenen RMA-S Zellen im Verhältnis 5:1 gemischt und-fünf Tage lang weiterkultiviert (Stuber et al., 1994). Die Zahl der überlebenden Effektor-Milzzellen in den verschiedenen Kulturen wurde bestimmt, dann wurden die Zellen für die Bestimmung der CTL-Aktivität mittels Standard 4 h - Europium-Freisetzungs-Assay (Blomberg et al., 1993) in verschiedenen Verhältnissen mit RMA-S Zellen vermischt, die zuvor mit dem Peptid ASNENMETM und mit Europium-Chelat versetzt worden waren. Wie Fig. 10 zeigt, wurde die spezifische Immunität in diesem Versuch nur durch Vakzinierung mit Peptid und fpLys erzielt, nicht jedoch, wenn das Peptid allein verabreicht wurde.

### Beispiel 9

### Testung verschiedener basischer Polyaminosäuren auf ihre Fähigkeit, die Internalisierung und/oder Bindung von Peptiden an APCs zu verstärken

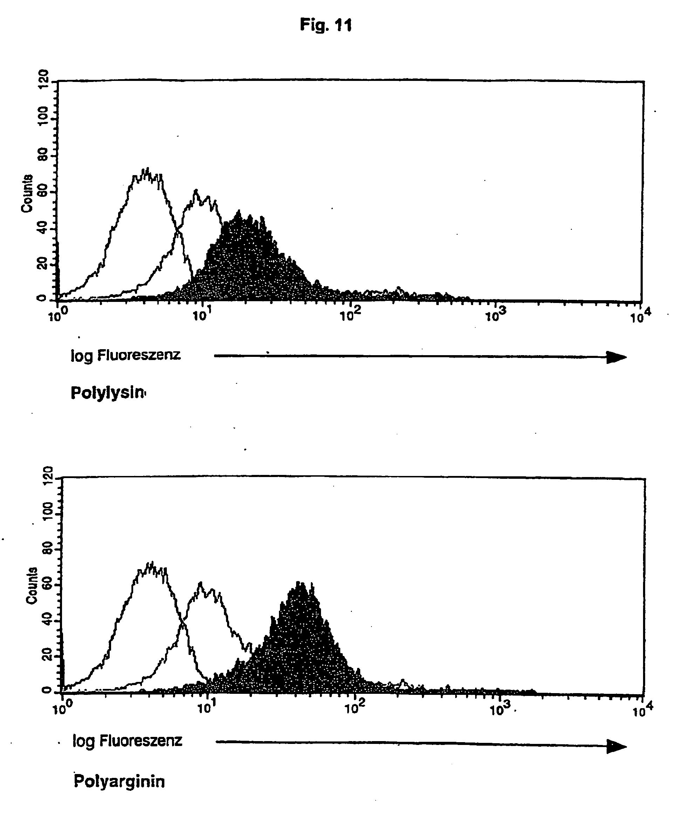

Für diese Tests wurde ein Fluoreszenzassay verwendet: Ein Modell-Peptidantigen der Sequenz LFEAIEGFI (MHC Kd-restringiert) wurde mit dem Fluoreszenzfarbstoff Fluoresceinisothiocyanat (FITC) nach Vorschrift des Herstellers (Molecular Probes) markiert. Die Aufnahme bzw. Bindung von FITC-markiertem Peptid allein ("pulsed") oder zusammen mit verschiedenen Konzentrationen von basischen Aminosäuren (Polylysin mit einer Kettenlänge von 16 bis 490, Polyarginin mit einer Kettenlänge von 15 bis 720) durch die MHC Kd-restringierte Monozyten-Makrophagen-Zellinie P388D1 wurde mittels Durchflußzytometrie gemessen. Dazu wurden 1 x 10⁶ P388D1-Zellen in einem Endvolumen von 1 ml Medium (DMEM/10 % FCS) in Zentrifugenröhrchen mit 5 µg FITC-markiertem Peptid allein oder mit einer Mischung aus Peptid und Polyaminosäure 30 min bei 37°C inkubiert und anschließend intensiv gewaschen, um freies Peptid zu entfernen. Die Polyaminosäuren wurden in einer Konzentration von 50, 25, 12, 6 und 3 µg pro ml Medium, enthaltend 5 µg FITC-markiertes Peptid zugesetzt. Die relative Fluoreszenzintensität der verschiedenen Proben wurde verglichen, um die Effizienz der Aufnahme und/oder Bindung des Peptids zu beurteilen. Das Ergebnis dieser Versuche ist in Fig. 11 dargestellt; die Versuche wurden mit jeweils 25 µg pL450 bzw. pArg450 durchgeführt. Unter den gewählten Bedingungen zeigte sich Polyarginin ca. fünfmal effizienter als Polylysin.

### Beispiel 10

### Untersuchung des Mechanismus, über den Peptide von APCs aufgenommen werden

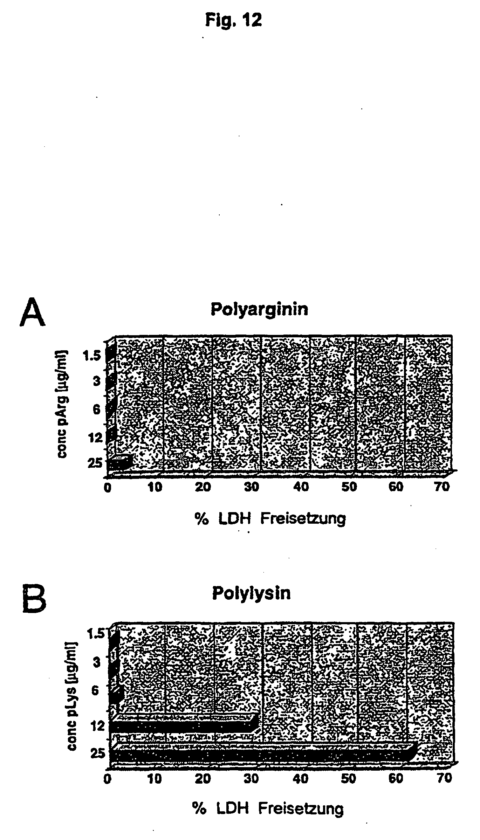

Peptide können von APCs durch spezifische Mechanismen wie Makropinozytose oder Rezeptor-vermittelte Endozytose (Lanzavecchia, 1996) aufgenommen werden. Ein alternativer Mechanismus kann darin bestehen, daß die Polyaminosäuren die Zellmembran durchlässig machen können und auf diese Weise die Diffusion von Peptiden vom Medium ins Zytoplasma ermöglichen.
a) Ob eine Permeabilisierung der Zellmembran stattfindet, wurde getestet, indem die Freisetzung des zytoplasmatischen Enzyms Lactatdehydrogenase (LDH) nach Inkubation von P388D1-Zellen mit Polyaminosäuren (Polylysin oder Polyarginin) unter isotonischen Bedingungen unter Verwendung des käuflich erhältlichen Kits (Cytotox 96, Promega, Madison, Wisconsin, USA) nach Vorschrift des Herstellers gemessen wurde. Aufgrund der in Fig. 12a erhaltenen Ergebnisse ist anzunehmen, daß die Wirkung von pLys darin besteht, daß es die Zellmembranen durchlässig macht, was sich in hohen Konzentrationen von unter isotonischen Bedingungen freigesetztem zytoplasmatischem Enzym äußert. Im Gegensatz dazu wurde nach pArg-Behandlung (Fig. 12b) praktisch kein LDH nachgewiesen. Beim Vergleich von Proben, die mit Polyaminosäuren allein behandelt worden waren, wurde im Vergleich zur Behandlung mit einer Mischung von Polylysin oder Polyarginin mit Peptid kein Unterschied in der LDH-Freisetzung festgestellt. Nach Inkubation mit Peptid allein wurde keine meßbare LDH-Aktivität nachgewiesen.
b) Ob eine Intemalisierung von FITC-markierten Peptiden in Gegenwart oder Abwesenheit von basischen Polyaminosäuren stattfindet, wurde auf der Grundlage des von Midoux et al., 1993, veröffentlichten Prinzips untersucht: Von Zellen internalisierte Partikel werden in Endosomen transportiert. Im Vergleich zum Zytoplasma oder Zellkultur-Medium, die neutrale pH-Werte aufweisen, sind diese Organellen mit einem pH-Wert von ca. 5 sauer. Die von FITC emittierte Fluoreszenz ist stark pH-abhängig. In einer Umgebung mit pH-Bedingungen, wie sie in Endosomen zu finden sind, wird die Fluoreszenz unterdrückt. Daher zeigen FITC-markierte Peptide, die von den Zellen in die Endosomen aufgenommen werden, eine verminderte Fluoreszenz. Bei Zugabe von Monensin wird der niedrige pH-Wert der Endosomen neutralisiert, was zu einer meßbaren verstärkten Fluoreszenz der internalisierten FITC-markierten Peptide führt.

Die Zellen wurden mit einer Mischung aus Polyarginin (durchschnittlicher Molekulargewichtsbereich 100.000, Kettenlänge 490) und Fluoreszenzmarkiertem Peptid bei 4°C oder 37°C inkubiert. Ein Aliquot der bei 37°C inkubierten Proben wurde zurückgehalten und vor der Durchflußzytometrieanalyse bei 4°C mit 50 µM Monensin behandelt.

Es zeigte sich, daß die Inkubation von APCs mit bestimmten basischen Polyaminosäuren wie Polylysin (pLys) und Polyarginin (pArg) die Aufnahme bzw. Bindung der Peptide an APCs verstärkt.

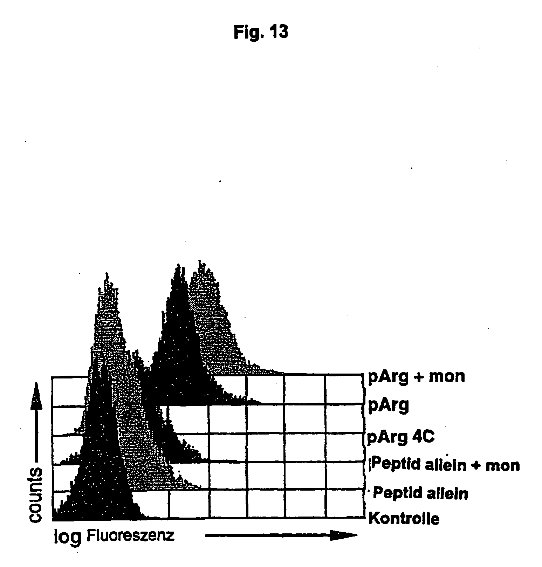

Wie sich aus Fig. 13 ergibt, wurde in Zellen, die mit Peptid allein und mit Monensin behandelt worden waren, nur ein geringer Anstieg der Fluoreszenz beobachtet. Im Gegensatz dazu waren die Fluoreszenzsignale in Proben, die mit Monensin und einer Mischung aus Polyarginin und Peptid behandelt worden waren, stark erhöht. Keine Peptidaufnahme wurde beobachtet, wenn die Proben bei 4°C inkubiert wurden. Ein signifikanter Anstieg der Fluoreszenz nach Monensinbehandlung weist darauf hin, daß die mit pArg bewirkte Beladung der Peptide dazu führt, daß diese in Vesikeln innerhalb der Zelle akkumuliert werden (Midoux et al., 1993; Fig. 13 ). Wie erwartet, wurde nach Monensin-Behandlung von Polylysin-beladenen Proben nur ein geringer Anstieg der Fluoreszenz beobachtet. Das Beladen mit Polylysin bei 4°C bewirkte einen meßbaren Anstieg der Fluoreszenz, was ein weiterer Hinweis dafür ist, daß die Wirkung von Polylysin hauptsächlich auf eine Permeabilisierung der Zellmembranen zurückzuführen ist (Fig. 12b).

### Beispiel 11

### Untersuchung der Beladung von APCs mit kurzen Peptiden

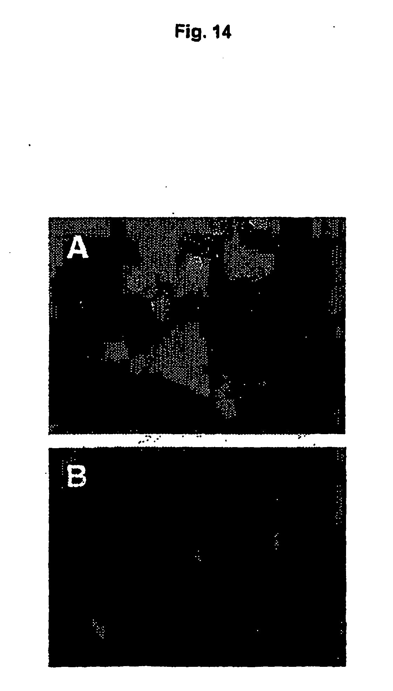

Aus Knochenmark stammende, mit GM-CSF erhaltene APCs wurden mit einer Kombination eines fiuoreszenzmärkierten Peptids plus Polylysin (pL200; Sigma) oder mit Peptid allein mittels Fluoreszenzmikroskopie untersucht. Für den mikroskopischen Nachweis der Peptidaufnahme wurden die APCs auf Objektträger ausgesät und mit 40 µg Fluorescein-markiertem Peptid LFEAIEGFI allein oder mit einer Mischung mit 50 µg/ml Polylysin (pL200) und 40 µg/ml Peptid LFEAIEGFI 30 min bei 37°C inkubiert. Nach ausgiebigem Waschen wurden die Zellen mit 4 % para-Formaldehyd fixiert, mit anti-Fadent (Dako, Glostrup, Dänemark) eingedeckt und fluoreszenzmikroskopiert (Zeiss). Die Kerne wurden mit 4.6-Diamidino-2-phenylindol (DAPI; Sigma) gegengefärbt. Wie in den Fluoreszenzmikrofotografien von Fig. 14 gezeigt wird, weisen die Zellen, die mit Peptid und Polylysin inkubiert worden waren (A), gegenüber den Zellen, die mit dem Peptid allein (B) behandelt worden waren, eine deutlich verstärkte Aufnahme des Peptids auf. Während die Fluoreszenz bei den Zellen, die nur mit Peptid allein behandelt ("pulsed") worden waren, nur vereinzelt und teilchenförmig auftrat, zeigte sich die intensive Fluoreszenz der in Gegenwart von Polylysin mit Peptid beladenen Zellen als nicht lokalisiert und im allgemeinen gleichmäßiger über die ganze Zelle verteilt.

### Beispiel 12

### Quantitative Bestimmung der Beladung von Zellen mit Peptid mittels Durchflußzytometrie ("Transloading Assay")

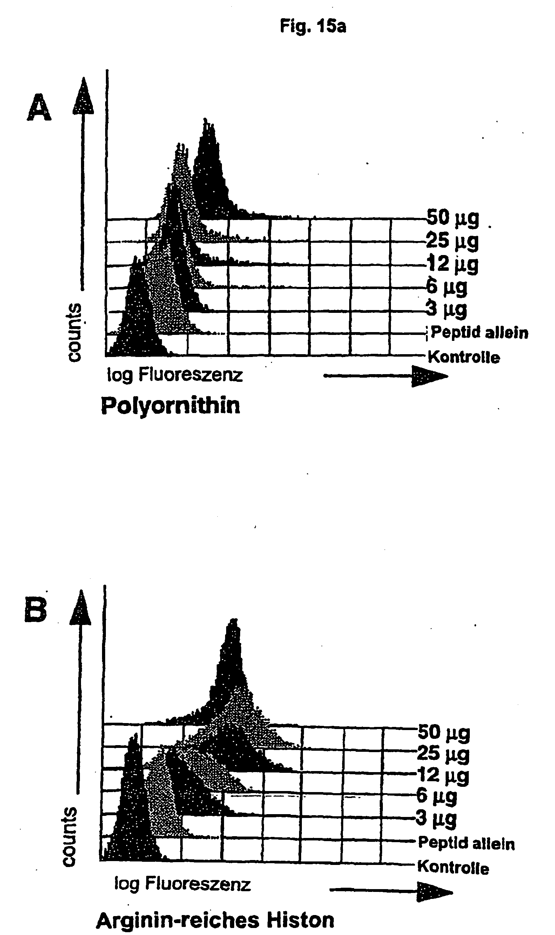

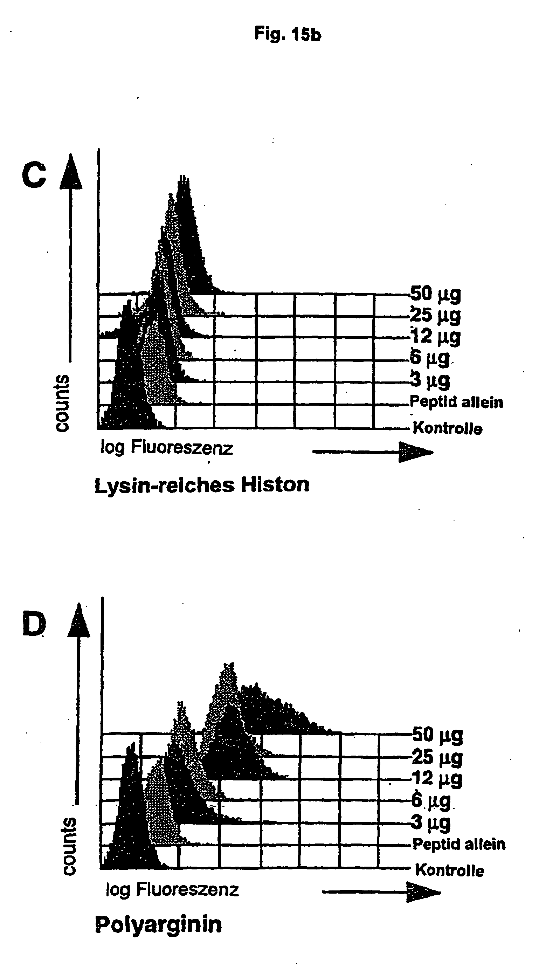

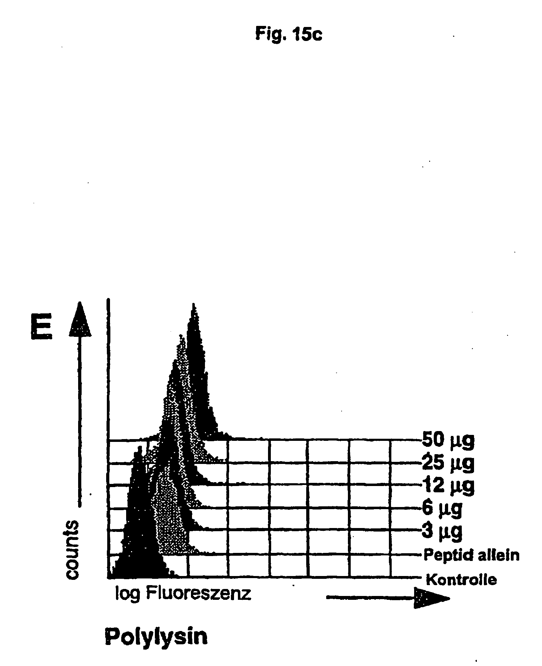

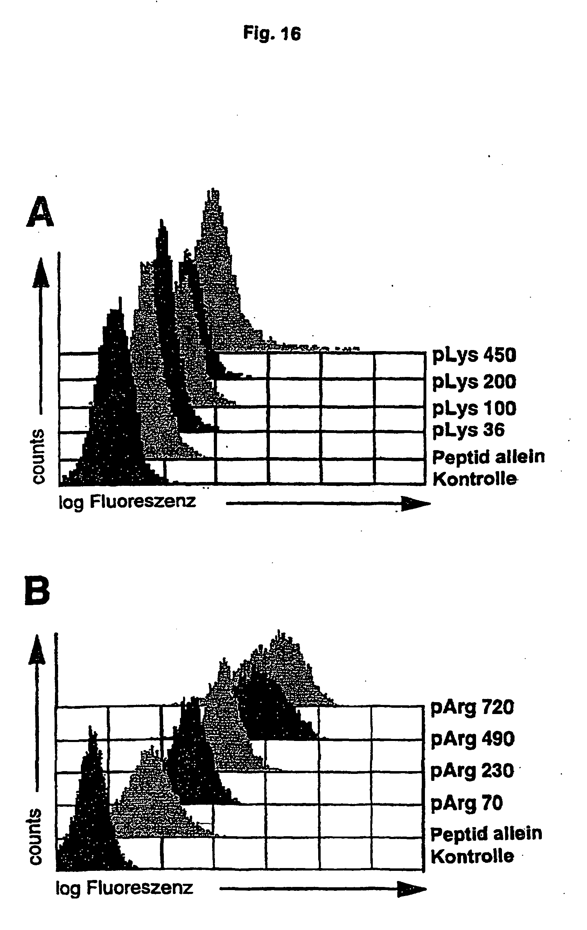

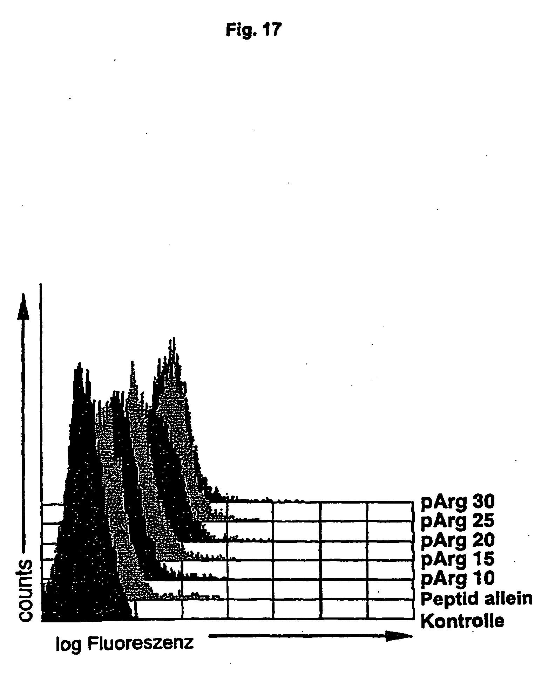

a) Nachdem sich in den im Beispiel 11 durchgeführten Versuchen gezeigt hatte, daß APCs für das Beladen mit kleinen Peptiden hervorragende Zielzellen sind, wurden *in vitro* FACS-Assays durchgeführt, um geeignete Adjuvantien für Peptid-Vakzine zu identifizieren. Dieser Assay erlaubt eine rasche quantitative Testung fluoreszenzmarkierter Peptide; als Modell-Peptid wurde das Peptid der Sequenz LFEAIEGFI verwendet. In diesen Versuchen wurden als APCs die Mauszellinie P388D1 verwendet. 1 x 10⁶ Zellen, wurden in einem Endvolumen von 1 ml DMEM-Medium mit hohem Glukosegehalt und 10 % FCS 30 min bei 37°C mit 5 µg Peptid bei einer Fluorescein-Endkonzentration von 5 nmol/ml inkubiert. Die Zellen wurden entweder mit Peptid allein oder mit einer Kombination von Peptid und Polykationen oder von Peptid und Histonen bei steigenden Konzentrationen (3 bis 50 µg/ml). wie in Fig. 15 angegeben, behandelt. Es wurden die folgenden Verbindungen verwendet: A: Polyomithin (durchschnittlicher Molekulargewichtsbereich 110,000, Kettenlänge 580); B: argininreiches Histon; C: lysinreiches Histon; D: Polyarginin (durchschnittlicher Molekulargewichtsbereich 100,000, Kettenlänge 490); E: Polylysin (durchschnittlicher Molekulargewichtsbereich 94,000, Kettenlänge 450). In Vorversuchen wurde ermittelt, daß eine Inkubationszeit von 30 min eine maximale Peptidaufnahme ergab. Eine längere Behandlung (4 bzw. 8 h) ergab keine signifikante Steigerung des Fluoreszenzsignals. Vor der Analyse wurden die Zellen 5 x mit einem großen Volumen PBS, enthaltend 0.2 % BSA, gewaschen. Die Zellen wurden in 1 ml eiskaltem PBS/0.2 % BSA wieder aufgenommen und mittels Durchflußzytometrie (FACScan; Becton Dickinson, San Jose, CA, USA) untersucht.
Polyarginin und Polylysin erwiesen sich als die wirksamsten Adjuvantien; Polyomithin zeigte unter den gewählten Bedingungen eine zytotoxische Wirkung. Die Verstärkung der Peptidaufnahme durch Polyarginin und Polylysin korreliert mit der Konzentration (Fig. 15 d, e); die Aufnahme nimmt mit der Kettenlänge zu (Fig. 16).
Es wurde beobachtet, daß Polyarginin mit einer Steigerung von 3 Zehnerpotenzen gegenüber der Kontrolle einen breiteren geeigneten Konzentrationsbereich aufweist als Polylysin, das eine maximale Steigerung von weniger als 2 Zehnerpotenzen zeigte, und daß es bei allen verwendeten Kettenlängen für den Transport von Proteinen wirkungsvoller ist, als Polylysin (Fig. 16): Polyarginin ermöglicht einen effizienten Transport bei so niedrigen Konzentrationen wie 3 µg/ml, während für Polylysin Konzentrationen von > 25 µg/ml erforderlich waren, um eine signifikante Steigerung der Fluoreszenz zu bewirken (Fig. 15 d,e).
b) Um festzustellen, ob es für den Peptidtransport eine untere Grenze der Kettenlänge gibt, wurden Polyarginine verschiedener Kettenlänge (10-30 Reste) synthetisiert und auf ihre Fähigkeit untersucht, den Peptidtransport bei hohen Konzentrationen der Polykationen zu steigern (Fig. 17). Für diese Versuche wurde das Peptid LFEAIEGFI verwendet, die getesteten Polyargininpolymeren wurden in einer Konzentration von 100 µg/ml eingesetzt.
Eine, wenn auch geringfügige, Steigerung des Peptidtransports wurde bereits mit dem kürzesten getesteten Polyarginin beobachtet.
c) Basische Aminosäuren sind positiv geladene Moleküle. Man kann daher annehmen, daß negativ geladene Peptide über elektrostatische Wechselwirkungen an diese Polykationen binden könnten, was möglicherweise zu einer verstärkten Peptidaufnahme führt. Um diese Hypothese zu testen, wurde die Fähigkeit kationischer Polyaminosäuren, kurze Peptide in Abhängigkeit ihrer Ladung in P388D1-Zellen aufzunehmen, verglichen. In nachstehender Tabelle sind die verwendeten negativ geladenen Peptide, die alle die für die MHC-I-bindung erforderlichen Bedingungen erfüllen (Rammensee et al., 1995) aufgelistet. Peptid 1 ist vom Maus-TRP ("tyrosinase related protein") abgeleitet, Peptid 2 stammt vom Influenza-Hämagglutinin (Schmidt et al., 1996), Peptid 3 von Maus-Tyrosinase, Peptid 4 vom P198-Tumorantigen, Peptid 5 von der beta-Galaktosidase (Gavin et al., 1993). ("Mr" steht für Molekulargewichtsbereich, "fluor" steht für Fluoreszein).

**Tabelle**

| Sequenz | Mr | Mr fluor | Ladung | Ladung + fluor |
|---|---|---|---|---|
| YAEDYEEL | 1031 | 1389 | 4 x negativ | 6 x negativ |
| LFEAIEGFI | 1038 | 1396 | 2 x negativ | 4 x negativ |
| IFMNGTMSQV | 1127 | 1485 | neutral | 2 x negativ |
| KYQAVTTTL | 1024 | 1382 | 1 x positiv | 1 x negativ |
| TPHPARIGL | 961 | 1319 | 2 x positiv | neutral |

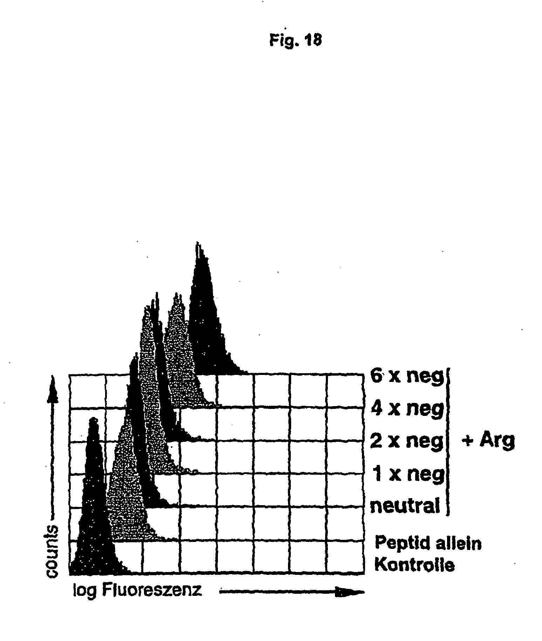

Aufgrund der zur Markierung des Peptids mit Fluoreszein verwendeten Methode werden zwei negative Ladungen eingeführt. Es zeigte sich, daß nach Inkubation mit Polyarginin die Peptide (5 nmol pro Probe) mit der höchsten Zahl an negativen Ladungen am effizientesten in P388D1-Zellen transportiert werden, was darauf hinweist, daß eine ionische Wechselwirkung zwischen Peptid und Polykation den Peptidtransport in Zellen noch weiter steigert (Fig. 18). Dennoch wurden im Vergleich zu Zellen, die mit Peptid allein behandelt wurden, auch im Fall neutraler Peptide in Gegenwart der Polykationen größere Mengen aufgenommen. Die Behandlung mit Peptid allein resultierte bei allen getesteten Peptiden in beinahe identischen Fluoreszenzsignalen; aus Darstellungsgründen wird in Fig.18 als "Peptid allein" stellvertretend das mit Peptid LFEAIEGFI erhaltene Fluoreszenzsignal gezeigt.

### LITERATUR

Alexander, J. et af., 1989, Immunogenetics 29, 380
Allred, D.C. et al.,1992, J. Clin. Oncol. 10 (4), 599-605
Avrameas, A. et al.,1996, Eur. J. Immunol. 26, 394-400
Behr, J.P., 1994, Bioconjug-Chem., Sept-Oct, 5(5), 382-9
Bertoletti, A. et al., 1994, Nature 369, 407-410
Biologic Therapy of Cancer, Editors: DeVita, V.T.Jr., Hellman, S., Rosenberg, S.A., Verlag J.B. Lippincott Company, Philadelphia, New York, London, Hagerstown
Blomberg, K. und Ulfstedt, A.C., 1993, J.Immunol. Methods 160:27-34
Boon, T., 1992, Adv Cancer Res 58.177-210
Boon, T., 1993, Spektrum der Wissenschaft (Mai), 58-66
Boon, T. et al., 1994, Annu. Rev. Immunol. 12, 337-65
Boon, T. und van der Bruggen, P., 1996, J Exp Med 183, 725-729
Braciale, T.J. und Braciale, V.L., 1991, Immunol. Today 12,124-129
Brocke, S. et al., 1996, Nature 379 (6563), 343-346
Bronte, et al., 1995, J. Immunol. 154, 5282
Carrel, S. und Johnson, J.P., 1993, Current Opinion in Oncology 5, 383-389
Coligan, J.E., et al., 1991, Nature 351, 290-296
Coligan, J.E., et al., 1991, Current Prot. in Immunol., Wiley, New York
Coulie, P.G. et al., 1992, Int. J. Cancer, 50, 289-297
Coulie, P. G. et al., 1995, Proc Natl Acad Sci U S A 92, 7976-80
Coulie, P.G. et al., 1994, J. Exp. Med. 180, 35-42
Cox, A.L. et al., 1994, Science 264, 5159, 716-9
Current Protocols im Molecular Biology, 1995, Herausgeber: Ausubel F.M., et al., John Wiley & Sons, Inc.
Dranoff, G. et al., 1993, Proc. Natl. Acad. Sci. USA 90, 3539-3543
Dranoff, G. und Mulligan, R.C., 1995, Advances in Immunology 58, 417
Falk, K. et al., 1991, Nature 351,290-296
Felgner, J.H. et al., 1994, J. Biol. Chem. 269, 2550-2561
Feltkamp, M.C. et al., 1995, Eur. J. Immunol. 25 (9), 2638-2642
Fenton, R.G. et al., 1993, J. Natl. Cancer Inst. 85, 96, 1294-302
Fisk, B. et al., 1995, J, Exp. Med. 1881, 2109-2117
Flow Cytometry, Acad. Press, Methods in Cell Biology, 1989, Vol. 33, Herausgeber: Darzynkiewicz, Z. und Crissman, H.A.
Gedde Dahl, T. et al., 1992, Hum Immunol. 33, 4, 266-74
Grohmann, U. et al., 1995, Eur. J. Immunol. 25, 2797-2802
Guarini, A. et al., 1995, Cytokines and Molecular Therapy 1, 57-64
Han, X.K. et al., 1995, PNAS 92, 9747-9751
Handbuch: FACS VantageTM User's Guide, April 1994, Becton Dickinson
Handbuch: CELL Quest TM Software User's Guide, June 1994, Becton Dickinson
Henderson, R. A., und Finn, O. J.. 1996, Advances in Immunology 62, 217-256
Hérin M. et al., 1987, Int. J. Cancer, 39, 390
Hock, H. et al., 1993, Cancer Research 53, 714-716
Houbiers, J. G., et al., 1993; Eur J Immunol 23, 2072-7.
Huang, A. Y. C., und Pardoll, D. M. (1996). Proc Natl Acad Sci U S A 93, 9730-5
Inaba, K., et al., 1992, J Exp Med 176, 1693-1702
Jung, S. et al., 1991, J. Exp. Med. 173, 1, 273-6
Kawakami, Y. et al., 1994, Proc. Natl. Acad. Sci. USA 91, 6458-62
Kawakami, Y. et al., 1994a, Proc. Natl. Acad. Sci. USA 91, 9, 3515-9
Kawakami, Y. et al., 1994b, J. Exp. Med. 180, 1, 347-52
Kawakami, Y. et al., 1995, The Journal of Immunol. 154, 3961-3968
Kärre, K. et al., 1986, Nature 319, 20. Feb., 675
Kersh, G.J. et al., 1996, Nature 380 (6574), 495-498
Kovacsovics Bankowski, M. und Rock, K.L., 1995, Science 267, 243-246
Lanzavecchia, A., 1996, Curr. Opin. Immunol. 8, 348-354
Lehmann, J.M. et al., 1989, Proc. Natl. Acad. Sci. USA 86, 9891-9895
Lethe, B. et al., 1992, Eur. J. Immunol. 22, 2283-2288
Li, H., et al., 1989, J Exp Med 169, 973-986
Lill, N. L., Tevethia, M. J., Hendrickson, W. G., und Tevethia, S. S. (1992). J Exp Med 176, 449-57
Ljunggren, H.G., et al., 1990, Nature 346:476-480
Loeffler, J.-P. et al., 1993, Methods Enzymol. 217, 599-618
Lopez, J.A., et al., 1993, Eur. J. Immunol. 23, 217-223
MacBroom, C.R. et al., 1972, Meth. Enzymol. 28, 212-219
Mackiewicz, A. et al., 1995, Human Gene Therapy 6,805-811
Malnati, M.S. et al., 1995, Science 267, 1016-1018
Mandelboim, O. et al., 1994, Nature 369, 5.May, 67-71
Mandelboim, O. et al., 1995, Nature Medicine 1, 11, 1179-1183
Marchand, M., et al., 1995, Int J of Cancer 63, 883-5
Mclntyre, C.A.,et al.,1996 Cancer Immunol.Immunother. 42:246-250.
Midoux, P., et al., 1993, NATO ASI Series H67, 49-64
Morishita, R. et al., 1993, J. Clin. Invest. 91, 6, 2580-5
Nabel, G.J. et al., 1993, Proc. Natl. Acad. Sci. USA 90,11307-11311
Noguchi, Y. et al., 1994. Proc Natl Acad Sci U S A 91, 3171-3175
Oettgen, H.F. und Old, L.J., 1991, Biologic Therapy of Cancer, Editors: DeVita, V.T.Jr., Hellman, S., Rosenberg, S.A., Verlag J.B. Lippincott Company, Philadelphia, New York, London, Hagerstown, 87-119
Ostrand-Rosenberg, S., 1994, Current Opinion in Immunology 6, 722-727
Pardoll, D.M., 1993, Immunology Today 14, 6, 310
Practical Immunology, Editors: Leslie Hudson and Frank C. Hay, Blackwell Scientific Publications, Oxford, London, Edinburgh, Boston, Melbourne
Peace, D.J. et al., 1991, J. Immunol. 146, 6, 2059-65
Peoples, G.E. et al., 1994, J. Immunol. 152.10,4993-9
Plautz, G.E. et al., 1993, Proc. Natl. Acad. Sci. USA 90, 4645-4649
Porgador, A., Gilboa, E., 1995, J. Exp. Med. 182, 255-260
Puccetti, P. et al., 1995, Eur. J. Immunol. 24, 1446-1452
Rammensee, H.G., et al., 1993, Annu Rev Immunol 11, 213-44
Rammensee, H.G. et al., 1993, Current Opinion in Immunology 5, 35-44
Rammensee, H.G., et al., 1995, Current Biology 7, 85-96
Rammensee, H.G., 1995, Current Opinion in Immunology 7, 85-96
Rammensee, H.G., et al., 1995, Immunogenetics 41, 178-228
Remington's Pharmaceutical Sciences, 18. Auflage 1990, Mack Publishing Company, Easton, Penn. 1990
Remy, J.S. et al., 1994, Bioconjug-Chem., Nov-Dec, 5(6), 647-54
Rennie, J. und Rusting, R., 1996, Scientific American September, 28-30
Rivoltini, L. et al., 1995, The Journal of Immunology 154, 5 2257-2265
Robbins, P.F., et al., 1994, Cancer Res 54, 3124-6
Robbins, P. F., et al., 1995, J Immunol 154, 5944-50
Robbins, P.F. und Rosenberg, S.A., 1996, Journ. Exp. Med. 183, 1185-92.
Robbins, P.F. und Kawakami, Y., 1996, Curr Opin Immunol 8, 628-636
Roitt I.M., Brostoff J., Male D.K. Immunology, Churchill Livingstone
Rosenberg, S. A., 1996, Annual Reviews of Medicine, 47, 481 - 491
Ryser, H.J. und Hancock, R., 1965, Science 150, 501-503
Ryser, H.J. und Shen, W.C., 1978, Proc Natl Acad Sci USA 75, 3867-3870
Schmidt, W., et al., May 1995, Proc. Natl. Acad. Sci. USA, 92,4711-4714
Schmidt, W., et al., 1996, Proc Natl Acad Sci USA, 93, 9759-63
Sette, A. et al., 1994, Mol.Immunol. 31(11):813-822,
Shen, W.C. und Ryser, H.J., 1978, Proc. Natl. Acad. Sci. USA, 75, 1872-1876
Shen, W.C. und Ryser, H.J., 1979, Mol. Pharmacol. 16, 614-622
Shen, W.C. und Ryser, H.J., 1981, Proc. Natl. Acad. Sci. USA, 78, 7589-7593
Skipper, J., und Stauss, H.J., 1993, J. Exp. Med. 177, 5, 1493-8
Slingluff, C.L. et al., 1994, Current Opinion in Immunology 6, 733-740
Stein, D. et al., 1994, EMBO Journal, 13, 6, 1331-40
Stuber, G. et al., 1994, Eur. J. Immunol 24, 765-768
Sykulev, Y. et al., 1994, Immunity 1, 15-22
Theobald, M., Levine, A. J., und Sherman, L. A. (1995) PNAS 92, 11993-7
Tibbets, L. M. et al., 1993, Cancer, Jan. 15., Vo1.71, 2, 315-321
Tykocinski, M.L. et al., 1996, Am. J. Pathol. 148, 1-16
van der Bruggen, P. et al., 1994, Eur. J. Immunol. 24, 9, 2134-40 Issn: 0014-2980
Van der Eynde, B. und Brichard, V.G., 1995, Current Opinion Immunol. 7, 674-81
Van Pel, A. und Boon, T., 1982, Proc. Natl. Acad. Sci. USA 79, 4718-4722
Van Pel, A., et al., 1995, Immunological Reviews 145,229-250
Vitiello, A. et al, 1995, J. Clin. Inv. 95, 1, 341-349
Wagner, E., et al., 1990, Proc Natl Acad Sci USA 87, 3410-4
Wagner, E., et al., 1992, Proc Natl Acad Sci USA 89, 6099-103
Wang, R.F., et al., 1995, J Exp Med 181, 799-804
Weynants, P. et al., 1994, Int. J. Cancer 56, 826-829
Widmann, C. et al., 1992, J. Immunol. Methods 155 (1), 95-99
Wölfel, T. et al., 1994 a), Int. J. Cancer 57, 413-418
Wölfel, T. et al., 1994 b), Eur. J. immunol. 24, 759-764
York, I.A. und Rock, K.L., 1996, Ann. Rev. Immunol. 14, 369-396
Yoshino, I. et al., 1994 a), J. Immunol. 152, 5, 2393-400
Yoshino, I. et al., 1994 b), Cancer Res., 54,13, 3387-90
Young, J.W., Inaba, K., 1996, J. Exp. Med., 183, 7-11
Zatloukal, K. et al., 1993, Gene 135,199-20
Zatloukal, K. et al., 1995, J. Immun. 154, 3406-3419

## Patentansprüche

1. Pharmazeutische Zusammensetzung, enthaltend
- mindestens ein immunmodulatorisch wirkendes Peptid, Protein oder Proteinfragment, ausgenommen Peptide, die von Tumoraneigenen des mit der Zusammensetzung zu behandelnden Patienten abgeleitet sind und an ein MHC-I- oder an ein MHC-II-Molekül des Patienten binden,
- zusammen mit einem Adjuvans, wobei das Adjuvans Polyarginin ist.

2. Pharmazeutische Zusammensetzung nach Anspruch 1, **dadurch gekennzeichnet, daß** das Peptid bzw. das zelluläre Abbauprodukt des Proteins oder Proteinfragments an mindestens ein MHC-Molekül bindet, das von dem zu behandelnden Individuum exprimiert wird.

3. Pharmazeutische Zusammensetzung nach Anspruch 2, **dadurch gekennzeichnet, daß** das Peptid bzw. das zelluläre Abbauprodukt des Proteins oder Proteinfragments an ein MHC-I-Molekül bindet.

4. Pharmazeutische Zusammensetzung nach Anspruch 2, **dadurch gekennzeichnet, daß** das Peptid bzw. das zelluläre Abbauprodukt des Proteins oder Proteinfragments an ein MHC-II-Molekül bindet.

5. Pharmazeutische Zusammensetzung nach einem der vorhergehenden Ansprüche, **dadurch gekennzeichnet, daß** sie ein Peptid enthält, das von einem Protein eines pathogenen Erregers abgeleitet ist.

6. Pharmazeutische Zusammensetzung nach Anspruch 5, **dadurch gekennzeichnet, daß** das Peptid von einem bakteriellen Protein abgeleitet ist.

7. Pharmazeutische Zusammensetzung nach Anspruch 5, **dadurch gekennzeichnet, daß** das Peptid von einem viralen Protein abgeleitet ist.

8. Pharmazeutische Zusammensetzung nach einem der Ansprüche 2 bis 7, **dadurch gekennzeichnet, daß** sie mehrere Peptide enthält, die sich **dadurch unterscheiden, daß** sie an unterschiedliche MHC-Subtypen des zu behandelnden Individuums binden.

9. Pharmazeutische Zusammensetzung nach einem der Ansprüche 2 bis 8, **dadurch gekennzeichnet, daß** sie ein oder mehrere Peptide enthält, die von einem natürlich vorkommenden immunogenen Protein bzw. einem zellulären Abbauprodukt davon abgeleitet sind.

10. Pharmazeutische Zusammensetzung nach einem der Ansprüche 2 bis 8, **dadurch gekennzeichnet, daß** sie ein oder mehrere Peptide enthält, die verschieden sind von Peptiden, die von natürlich vorkommenden immunogenen Protein(en) oder Tumorantigen(en) bzw. eine zelluläre Abbauprodukt(en) davon abgeleitet sind.

11. Pharmazeutische Zusammensetzung nach Anspruch 1, **dadurch gekennzeichnet, daß** das Peptid ein Antagonist eines Peptids ist, das von einem Protein abgeleitet ist, das eine Autoimmunerkrankung verursacht.

12. Pharmazeutische Zusammensetzung nach einem der Ansprüche 1 bis 11, **dadurch gekennzeichnet, daß** sie als Lösung oder Suspension des Peptids und Polyarginin in einem pharmazeutisch annehmbaren Träger vorliegt.

13. Pharmazeutische Zusammensetzung nach einem der Ansprüche 1 bis 12 für die topische Verabreichung.

14. Pharmazeutische Zusammensetzung nach Anspruch 13 in Form eines Hydrogels.

## Claims

1. Pharmaceutical composition containing
- at least one peptide, protein or protein fragment with an immunomodulatory activity, with the exception of peptides that are derived from tumour antigens from the patient who is to be treated with the composition and that bind to an MHC-I or to an MHC-II molecule of the patient,
- together with an adjuvant, the adjuvant being polyarginine.

2. Pharmaceutical composition according to claim 1, **characterised in that** the peptide or the cellular breakdown product of the protein or protein fragment binds to at least one MHC molecule which is expressed by the individual to be treated.

3. Pharmaceutical composition according to claim 2, **characterised in that** the peptide or the cellular breakdown product of the protein or protein fragment binds to an MHC-I molecule.

4. Pharmaceutical composition according to claim 2, **characterised in that** the peptide or the cellular breakdown product of the protein or protein fragment binds to an MHC-II molecule.

5. Pharmaceutical composition according to one of the preceding claims, **characterised in that** it contains a peptide which is derived from a protein of a pathogenic agent.

6. Pharmaceutical composition according to claim 5, **characterised in that** the peptide is derived from a bacterial protein.

7. Pharmaceutical composition according to claim 5, **characterised in that** the peptide is derived from a viral protein.

8. Pharmaceutical composition according to one of claims 2 to 7, **characterised in that** it contains a plurality of peptides which differ **in that** they bind to different MHC-subtypes of the individual to be treated.

9. Pharmaceutical composition according to one of claims 2 to 8, **characterised in that** it contains one or more peptides which are derived from a naturally occurring immunogenic protein or a cellular breakdown product thereof.

10. Pharmaceutical composition according to one of claims 2 to 8, **characterised in that** it contains one or more peptides which are different from peptides derived from naturally occurring immunogenic protein(s) or tumour antigen(s) or cellular breakdown product(s) thereof.

11. Pharmaceutical composition according to claim 1, **characterised in that** the peptide is an antagonist of a peptide which is derived from a protein that causes an autoimmune disease.

12. Pharmaceutical composition according to one of claims 1 to 11, **characterised in that** it takes the form of a solution or suspension of the peptide and polyarginine in a pharmaceutically acceptable carrier.

13. Pharmaceutical composition according to one of claims 1 to 12 for topical application.

14. Pharmaceutical composition according to claim 13 in the form of a hydrogel.

## Revendications

1. Composition pharmaceutique contenant
- au moins un peptide, une protéine ou un fragment de protéine à effet immunomodulateur, à l'exception des peptides qui sont dérivés d'antigènes tumoraux du patient à traiter avec la composition et qui se lient à une molécule du CMH-I ou du CMH-II du patient,
- en même temps qu'un adjuvant, où l'adjuvant est la polyarginine.

2. Composition pharmaceutique selon la revendication 1 **caractérisée en ce que** le peptide ou le produit de dégradation cellulaire de la protéine ou du fragment de protéine se lie à au moins une molécule du CMH qui est exprimée par l'individu à traiter.

3. Composition pharmaceutique selon la revendication 2 **caractérisée en ce que** le peptide ou le produit de dégradation cellulaire de la protéine ou du fragment de protéine se lie à une molécule du CMH-I.

4. Composition pharmaceutique selon la revendication 2 **caractérisée en ce que** le peptide ou le produit de dégradation cellulaire de la protéine ou du fragment de protéine se lie à une molécule du CMH-II.

5. Composition pharmaceutique selon l'une des revendications précédentes **caractérisée en ce qu'**elle contient un peptide qui est dérivé d'une protéine d'un agent pathogène.

6. Composition pharmaceutique selon la revendication 5 **caractérisée en ce que** le peptide est dérivé d'une protéine bactérienne.

7. Composition pharmaceutique selon la revendication 5 **caractérisée en ce que** le peptide est dérivé d'une protéine virale.

8. Composition pharmaceutique selon l'une des revendications 2 à 7 **caractérisée en ce qu'**elle contient plusieurs peptides qui diffèrent par le fait qu'ils se lient à des sous-types du CMH de l'individu à traiter qui sont différents.

9. Composition pharmaceutique selon l'une des revendications 2 à 8 **caractérisée en ce qu'**elle contient un ou plusieurs peptides qui sont dérivés d'une protéine immunogène naturelle ou d'un produit de dégradation cellulaire de celle-ci.

10. Composition pharmaceutique selon l'une des revendications 2 à 8 **caractérisée en ce qu'**elle contient un ou plusieurs peptides qui sont différents de peptides qui sont dérivés d'une ou plusieurs protéines immunogènes naturelles ou d'un ou plusieurs antigènes tumoraux ou d'un ou plusieurs produits de dégradation cellulaire de ceux-ci.

11. Composition pharmaceutique selon la revendication 1 **caractérisée en ce que** le peptide est un antagoniste d'un peptide qui est dérivé d'une protéine qui provoque une maladie auto-immune.

12. Composition pharmaceutique selon l'une des revendications 1 à 11 **caractérisée en ce qu'**elle est présente sous forme de solution ou de suspension du peptide et de polyarginine dans un support pharmaceutiquement acceptable.

13. Composition pharmaceutique selon l'une des revendications 1 à 12 pour l'administration topique.

14. Composition pharmaceutique selon la revendication 13 sous forme d'un hydrogel.
